# FASCICULE DE BREVET EUROPEEN

(11) **EP 3 721 438 B1**
(45) Date de publication et mention de la délivrance du brevet: **12.01.2022**
(21) Numéro de dépôt: 18814906.6
(22) Date de dépôt: 07.12.2018
(51) Int. Cl.: G16H 30/40, G06Q 10/10, G06T 11/00, G16H 80/00, G06T 11/60

(54) **SYSTÈME ET PROCEDE DE TRAITEMENT D'IMAGES COLLABORATIF ET INTERACTIF**
SYSTEM UND VERFAHREN ZUR GEMEINSAMEN UND INTERAKTIVEN BILDVERARBEITUNG
SYSTEM AND METHOD FOR COLLABORATIVE AND INTERACTIVE IMAGE PROCESSING

(30) Priorité: 08.12.2017 FR 1771328
(43) Date de publication de la demande: 14.10.2020
(73) Titulaire: HEWEL S.A.S., 76100 Rouen (FR)
(72) Inventeur: LE NAOUR, Gilles, 94360 Bry-Sur-Marne (FR); LIEVAL, Fabien, 77600 Bussy-Saint-Georges (FR)
(74) Mandataire: Corret, Hélène
(86) Numéro de dépôt international: PCT/EP2018/084060
(87) Numéro de publication internationale: WO 2019/110834

(56) Documents cités:
- US-A1- 2014 292 814
- LENA MAIER-HEIN ET AL: "Can masses of non-experts train highly accurate image classifiers? A crowdsourcing approach to instrument segmentation in laparoscopic images", MEDICAL IMAGE COMPUTING AND COMPUTER-ASSISTED INTERVENTION - MICCAI 2014. 17TH INTERNATIONAL CONFERENCE, BOSTON, MA, USA, SEPTEMBER 14-18, 2014, PROCEEDINGS, PART II. II, LNCS 8674., vol. 17, 18 septembre 2014 (2014-09-18), pages 438-445, XP055556632, Germany
- LENA MAIER-HEIN ET AL: "Crowdtruth validation: a new paradigm for validating algorithms that rely on image correspondences", INTERNATIONAL JOURNAL OF COMPUTER ASSISTED RADIOLOGY AND SURGERY, vol. 10, no. 8, 18 avril 2015 (2015-04-18) , pages 1201-1212, XP055557447, DE ISSN: 1861-6410, DOI: 10.1007/s11548-015-1168-3
- JANET L. BAILEY ET AL: "Telementoring: using the Kinect and Microsoft Azure to save lives", INTERNATIONAL JOURNAL OF ELECTRONIC FINANCE, vol. 7, no. 1, 28 janvier 2013 (2013-01-28), page 33, XP055557480, ISSN: 1746-0069, DOI: 10.1504/IJEF.2013.051755
- Anonymous: "Console système - Wikipédia", , 22 octobre 2017 (2017-10-22), XP055557260, Extrait de l'Internet: URL:https://fr.wikipedia.org/w/index.php?t itle=Console_système&oldid=141754871 [extrait le 2019-02-14]

## Description

La présente invention concerne un système et un procédé de traitement d'images collaboratif et interactif, par exemple dans le cadre d'un contrôle de qualité industriel ou dans le cadre de l'assistance au diagnostic médical ou d'enseignement. L'invention est caractérisée dans les revendications.

L'invention s'applique notamment au domaine industriel (ex. aéronautique ou micro-électronique), au domaine médical et à tout autre domaine nécessitant un partage d'avis et d'analyse graphique d'images pour limiter les risques d'erreur d'appréciation individuels.

Dans de nombreux domaines techniques, l'analyse de données graphiques sous forme d'images nécessite d'avoir plusieurs avis et/ou de faire participer plusieurs experts pour assurer une analyse de bonne qualité des données et pouvoir poser, par la suite, un diagnostic pertinent. On pense bien entendu au domaine médical et particulièrement à l'imagerie médicale radiologique ainsi que les lames numériques des examens histologiques, dans lequel l'analyse précise et de bonne qualité de l'observation est essentielle pour pouvoir ensuite poser le bon diagnostic et proposer le bon traitement.

On pense également au domaine aéronautique dans lequel des analyses de structures doivent régulièrement être menées pour s'assurer que telle ou telle pièce ou assemblage n'a pas subi une déformation ou un traitement incompatible avec son maintien dans l'aéronef.

Il est alors essentiel de pouvoir s'assurer que le ou les spécialistes en charge de ces analyses ont bien repéré sur les images des structures les zones d'intérêt, comme des cellules cancéreuses dans le domaine médical ou des micro fissures dans le domaine aéronautique, et leur étendue.

L'invention s'applique également au domaine de l'enseignement pour permettre à un professeur d'enseigner à des étudiants l'analyse de certaines images (médicales ou industrielles) et d'évaluer ces mêmes étudiants sur des critères techniques objectifs. L'invention vise à permettre également aux étudiants en présentiel (c'est-à-dire ceux qui sont présents en classe, par opposition aux étudiants à distance), de questionner facilement leur professeur sur des zones particulières de l'image, en mode synchrone (c'est-à-dire en même temps) ou asynchrone (c'est-à-dire à des moments différents de la séance de travail).

Un exemple de domaine d'application plus particulier de l'invention est celui de l'Anatomie Cytologie pathologiques (ci-après « ACP »).

Il s'agit d'une spécialité médicale qui consiste à rechercher des anomalies tissulaires et cellulaires sur prélèvement effectué sur un organe. L'examen repose sur l'étude macroscopique et/ou microscopique de tissus, ou leurs dérivés, prélevés sur des patients. L'observation de lames histologiques tissulaires ou cellulaires (ci-après « lames ») est réalisée par un médecin anatomo-cytopathologiste (ou « médecin ACP ») qui liste, quantifie et qualifie ses observations dans un rapport. Ce dernier est transmis au médecin traitant ou clinicien qui va l'interpréter en prenant en compte d'autres données médicales issues d'autres examens, soit par exemple pour demander un contrôle chirurgical, soit pour proposer directement une chirurgie sur la base du premier examen biopsique, soit pour poser directement un diagnostic et proposer un traitement.

L'ACP est notamment utilisée dans la cadre du diagnostic des tumeurs et dans la prise en charge thérapeutique personnalisée et le traitement ciblé des cancers ou de certaines maladies non cancéreuses, mais également pour le diagnostic des maladies de cause inflammatoire, dégénérative, métabolique ou infectieuse.

Face à l'accroissement du nombre d'actes d'ACP, à la baisse de la démographie de médecins ACP et à l'émergence d'une exigence de traçabilité des actes d'ACP, le concept de « Digital Pathology » est apparu dans le courant des années 90, combinant l'ACP avec les technologies de numérisation à haut débit.

Ainsi, le développement des scanners de lames a permis l'émergence de cette discipline nouvelle qui est devenue un outil d'examen médical et de recherche, ainsi qu'un outil pédagogique performant.

De façon connue, les scanners de lames produisent des lames virtuelles (ci-après « lames virtuelles ») qui sont des images numériques qualifiées de « haute définition », car présentant un grand nombre de pixels (ex. de 10000 x 10000 pixels à 200000 x 200000 pixels), ce qui se traduit par un nombre d'octets très important, typiquement de l'ordre de plusieurs dizaines de mégaoctets à plusieurs gigaoctets (ex. de 10 Mo à 10 Go).

L'utilisation des lames virtuelles en ACP peut servir plusieurs usages complémentaires pour les praticiens (médecins ACP, chercheurs, experts médicaux) :
a) Usage individuel : un praticien pose un diagnostic médical sur un / des cas, ou consulte un / des cas types provenant de bibliothèques de cas pour se forger une opinion ;
b) Usage collectif : un praticien soumet un / des cas à ses pairs pour avis (ex. équipes médicales ou comités de relecture en ACP), ou forme un groupe d'autres praticiens autour de cas d'apprentissage (ex. enseignements universitaires et post-universitaires), ou conduit des projets de recherche associant ses pairs relativement à des lames spécifiques qui peuvent aboutir à des publications scientifiques.

Pour tous les usages collectifs, il est très souvent constaté le besoin de partage ou de recueil ou de traçabilité d'informations, ou d'organisation du travail de manière collaborative. Ce besoin n'est cependant pas rempli par le matériel et/ou les logiciels actuels.

En effet, plusieurs sociétés ont développé des logiciels spécialisés permettant la lecture et l'analyse des lames virtuelles ainsi que parfois l'aide au diagnostic.

Ces logiciels présentent tous les mêmes fonctionnalités : création de répertoires d'images de lames virtuelles, visualisation et manipulation d'image individuelles, définition de région d'intérêt (ci-après «ROI» pour region of interest en anglais) ou d'objet d'intérêt (ci-après « OOI » pour object of interest en anglais), comptage et annotations (c'est-à-dire commentaires de l'image, des OOI ou des ROI). Certains d'entre eux proposent également des solutions de reconnaissances automatiques de formes et de comptage, basées sur des algorithmes mathématiques.

Leur principal intérêt est de faciliter le stockage et la diffusion des lames virtuelles, d'éviter leur perte ou leur endommagement et le gain de temps pour annoter les images et compter les ROI/OOI.

Cependant, ces logiciels sont aujourd'hui destinés à une utilisation individuelle pour des usages individuels, depuis les ordinateurs des praticiens. Ils sont également généralement perçus comme complexes à utiliser et peu ergonomiques.

Le stockage des lames virtuelles, et des images en général, se fait quant à lui soit sur les ordinateurs, soit sur des serveurs accessibles à distance par des moyens de télécommunication (ex. hébergement cloud). Compte tenu du poids en octets des lames virtuelles, les équipements informatiques utilisés (ordinateur et serveur) doivent être dotés de ressources matérielles importantes, tant en puissance de calcul qu'en débit de transmission. Ces logiciels sont donc dépendants des architectures informatiques très hétérogènes des praticiens et des réseaux de télécommunication disponibles eux-mêmes très variables et inconstants. En pratique, il est souvent constaté un manque de fluidité dans l'affichage et la manipulation des lames virtuelles (ex. temps de latence), ce qui pénalise la qualité d'observation des lames virtuelles et donc constitue un premier enjeu important de l'invention.

En outre, ces logiciels ne sont ni collaboratifs ni interactifs. Ils ne proposent aucune solution pour assurer à la fois la traçabilité des divergences et leur analyse graphique entre les examens de plusieurs médecins ACP, ni résoudre le problème d'anonymisation des données.

Or, actuellement, dans le domaine médical comme dans le domaine aéronautique, il est courant de faire appel à des réseaux d'experts concernant l'interprétation d'images médicales ou d'assemblages de pièces mécaniques, dans les cas les plus complexes. Le diagnostic individuel initial s'enrichit alors de l'échange avec des pairs, jusqu'à aboutir à un diagnostic contradictoire basé sur plusieurs avis d'experts. Cependant, ces réseaux, souvent nationaux ou internationaux, sont interrogés à distance, et chaque expert travail seul et quand il est disponible.

De plus, dans le domaine médical, les exigences en matière d'anonymisation des données rendent la gestion du partage d'information complexe. Ainsi, il faut transmettre à chaque expert des données (prélèvement, image du prélèvement, âge, sexe du patient, etc.) anonymisées pour qu'il ne puisse pas identifier le patient, en lui fournissant avec les données un code d'identification. Il émet alors un rapport avec ce code d'identification, rapport qui doit ensuite être renvoyé au médecin traitant avec la bonne identité du patient. Cette chaîne d'anonymisation/réidentification est source d'erreurs humaines et doit comprendre de nombreux points de contrôle. En outre, cette chaîne est d'autant plus complexe que le nombre d'experts consultés est grand. Cela aboutit, pour limiter les risques, à ne consulter qu'un petit nombre d'experts, c'est-à-dire deux ou trois.

Malgré tout, il n'est pas rare que les résultats d'un patient soient annoncés à un autre patient, avec toutes les conséquences négatives que cela implique.

Le manque d'interaction entre les membres du réseau et les problèmes d'anonymisation des données constituent un deuxième enjeu de l'invention.

Par ailleurs, ces logiciels proposent des outils d'annotation textuelle d'images dont la définition des termes et la mise en œuvre sont laissées à la discrétion des praticiens. Plusieurs praticiens, consultant les mêmes images, peuvent ainsi définir des catégories d'OOI ou de ROI et des modules d'annotations hétérogènes, qui peuvent conduire in fine à des diagnostics différents.

On peut citer, par exemple, le document US20140292814 qui décrit un système d'aide au diagnostic à partir de lames virtuelles, dans lequel plusieurs praticiens peuvent ajouter sur une image de lame virtuelle des commentaires textuels issus de leur analyse cognitive de l'image. Il est ainsi possible d'ajouter un commentaire dans une zone de texte qui pointe sur un objet donné de l'image grâce à un trait qui relie la zone de texte à l'objet de l'image, exactement à la manière d'un traitement de texte dans lequel plusieurs auteurs peuvent ajouter des commentaires dans la marge, ces commentaires étant reliés à un mot du texte.

L'intérêt invoqué de ce système est que tous les commentaires peuvent être affichés simultanément au-dessus de l'image, c'est-à-dire juxtaposés et de manière regroupée en fonction de la localisation de l'objet commenté. Le lecteur est donc simplement en mesure d'identifier l'auteur (ou sa fonction) selon le format d'affichage de la zone de texte, mais il est incapable de déduire directement de l'image affichée la signification des commentaires.

En effet, d'une part ces commentaires sont simplement surimposés sur l'image qui n'est pas modifiée en tant que telle.

Certes les pixels de l'image affichés à l'écran sont modifiés pour pouvoir afficher les commentaires, mais les pixels du fichier image en tant que tels ne sont pas modifiés. Ainsi, les commentaires sont affichés en sur impression, c'est-à-dire juxtaposés à l'image sur l'écran et non insérés dans le fichier de l'image elle-même. C'est d'ailleurs démontré par le mode de réalisation dans lequel les commentaires n'apparaissent que lorsque le curseur est placé sur une zone commentée.

Autrement dit, les annotations dans ce document ne sont constituées que de commentaires en suraffichage dans des zones de texte et au format numérique texte alors que l'image est au format numérique image. Ces annotations ne sont pas des annotations graphiques de l'image, c'est-à-dire des modifications au format numérique image des pixels de l'image.

Ainsi, dans ce document, les commentaires et l'image ne sont donc pas au même format numérique.

D'autre part, le système de ce document ne peut analyser les commentaires puisqu'il s'agit de textes devant faire l'objet d'une interprétation sémantique par le lecteur, et que cette fonction est irréalisable automatiquement par un algorithme.

Conséquemment, puisque ce système ne peut analyser sémantiquement les commentaires et en déduire leur sens, il ne peut générer une synthèse de ces commentaires sous la forme, par exemple, d'un seul commentaire regroupant des opinions similaires et de différents commentaires avec des opinions différentes. L'utilisateur final doit donc obligatoirement lire tous les commentaires qui sont juxtaposés et surimposés à l'image lors de l'affichage (l'image en tant que telle n'est pas intrinsèquement modifiée), les interpréter sémantiquement et en faire une synthèse intellectuellement.

Ainsi, un tel système est d'autant moins utilisable que le nombre d'auteurs et/ou d'objets à commenter est élevé, car l'affichage devient illisible et l'interprétation longue et fastidieuse.

En outre, le système du document US20140292814 laissant chaque auteur libre de la rédaction de ses commentaires, notamment en termes de vocabulaire utilisé, il est rigoureusement impossible d'interpréter automatiquement ces commentaires.

Le document "Telementoring: using the Kinect and Microsoft Azure to save lives", Janet L. Bailey, Bradley K. Jensen; International Journal of Electronic Finance, 7(1), 2013, est considéré comme l'état de la technique le plus proche de l'objet de la revendication 1. Ce document divulgue un système de traitement d'image collaboratif et interactif (CAMI). En utilisant ce système, les experts sont aussi libre de la rédaction des annotations et peuvent utiliser des marques d'annotations de leur choix.

Les documents:
- "Can masses of non-experts train highly accurate image classifiers? A crowdsourcing approach to instrument segmentation in laparoscopic images", Maier-Hein L. et al., Med Image Comput Comput Assist Interv. 2014;17(Pt 2): 438-45, et
- "Crowdtruth validation: a new paradigm for validating algorithms that rely on image correspondences.", Maier-Hein L et al., Int J Comput Assist Radiol Surg. 2015 Aug; 10(8): 1201-12,
divulguent des systèmes pour le domaine de la chirurgie assistée par ordinateur. Ces systèmes utilisent la technique du "crowdsourcing" afin d'acquérir un grand nombre d'annotations d'images de référence qui peuvent servir comme des données d'entraînement et de validation de nouveaux algorithmes.

Permettre une analyse automatique et une synthèse automatique de cette analyse est donc un enjeu de l'invention.

Guider les utilisateurs dans l'annotation graphique des images est un autre enjeu de l'invention.

La présente invention vise donc à proposer un outil collaboratif et interactif destiné au travail présentiel, de manière synchrone ou asynchrone, de plusieurs personnes, et permettant aux participants d'examiner ensemble des images identiques et de partager les points de vue et les examens individuels des images, en utilisant des référentiels terminologiques et des outils d'annotation graphique associés à des séquences de décision identiques et pré-enregistrés spécifiques au contexte (par exemple de la pathologie).

Par collaboratif, on entend que plusieurs personnes peuvent étudier une même image simultanément, et participer à son analyse et à son commentaire.

Par interactif on entend que plusieurs personnes peuvent participer au traitement (analyse et marquage) d'une même image, c'est-à-dire que plusieurs personnes peuvent agir simultanément ou non, indépendamment ou en interaction, sur l'image pour la modifier ou l'annoter.

Par annotation graphique on entend une modification des pixels de l'image, et donc des données de l'image, cette annotation n'ayant aucun sens sémantique.

La présente invention vise également à permettre de comparer automatiquement les résultats d'examen graphique des experts, d'analyser les points communs et les différences graphiques pour pouvoir établir automatiquement un prérapport normé, assurant ainsi la traçabilité de l'ensemble de ces données, et comprenant une analyse statistique de ces données graphiques.

La présente invention vise également à permettre à l'un des experts, désigné comme auteur, de compléter ce prérapport avec son interprétation de l'image, éclairée par les échanges avec les autres intervenants.

L'auteur peut ainsi, grâce à ce rapport, transmettre aux responsables (médecins référents, chef d'équipe, etc.) qui, eux, poseront un diagnostic ultérieurement, une opinion éclairée du cas, c'est-à-dire en toute connaissance des convergences et des divergences d'analyse des experts, ainsi que de leur représentativité au sein du panel d'experts ayant analysé la ou les images.

La présente invention vise donc à proposer un outil collaboratif et interactif d'aide au diagnostic, mais à aucune étape le système ou le précédé selon l'invention n'est en mesure d'établir lui-même un diagnostic.

À cette fin, la présente invention propose un système de traitement d'image collaboratif et interactif comprenant une console de distribution d'images à traiter et de collecte d'images annotées apte à communiquer localement avec au moins deux interfaces de traitement aptes à afficher une image à traiter, et dans lequel la console et les interfaces sont programmées pour transmettre une copie de l'image à traiter sur chaque interface de traitement depuis la console de distribution, pour fournir des outils de modification graphique de la copie de l'image sur chaque interface pour obtenir une image annotée avec des modifications graphique, pour transmettre chaque image annotée sur la console de distribution depuis chaque interface de traitement, et pour générer une image résultat à partir des images annotées à l'aide d'un algorithme combinatoire analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes

Par algorithme combinatoire on entend que l'algorithme évalue la similitude des annotations graphiques des images annotées, fusionne les annotations graphiques par groupe de similitudes avant de les insérer dans l'image résultat sous un format unique par groupe, directement identifiable sur l'image résultat. En d'autres termes, un algorithme combinatoire selon l'invention analyse les données de l'image, synthétise cette analyse en fabriquant une seule image résultant de cette analyse, puis affiche le résultat de cette synthèse, de sorte que l'utilisateur en déduit directement la signification. La console et les interfaces sont programmées pour permettre un affichage selon un mode individuel dans lequel l'affichage de chaque interface est indépendant des autres interfaces, et pour permettre un affichage selon un mode commandé dans lequel l'affichage d'une première interface commande l'affichage sur les autres interfaces, le passage entre le mode individuel et le mode commandé étant réversible.

Selon d'autres modes de réalisation, qui peuvent être combinés entre eux :
- la console et les interfaces peuvent être programmées pour permettre de marquer sur la copie de l'image à traiter au moins une région d'intérêt définie par un identifiant unique, une forme représentative et des coordonnées dans la copie de l'image à traiter ;
- le système peut comprendre :
   - Une console de distribution et de collecte de données comprenant :
      ▪ un émetteur/récepteur sans fil d'un signal sans-fil local ;
      ▪ une mémoire de stockage pour au moins une image à traiter et pour au moins une image résultat ;
      ▪ une unité centrale programmée ;
   - au moins deux interfaces de traitement comprenant :
      ▪ un émetteur/récepteur sans fil d'un signal sans-fil local compatible avec l'émetteur/récepteur de la console pour permettre un échange local de données via le signal sans fil ;
      ▪ une mémoire de stockage pour au moins une image à traiter et pour au moins une image annotée ;
      ▪ une unité centrale programmée ;
- l'unité centrale de la console et l'unité centrale de chaque interface peuvent être programmées pour :
   - transmettre une copie d'au moins une image à traiter à toutes les interfaces ;
   - transmettre une copie d'au moins une image à traiter à toutes les interfaces ;
   - afficher une image à traiter entière sur chaque interface,
   - agrandir et/ou rétrécir sur commande l'image affichée et mémoriser une valeur de zoom représentative de l'agrandissement de l'image affichée par rapport à l'image entière ainsi qu'une position de l'image affichée par rapport à l'image entière ;
   - exécuter, à partir d'une première interface, un affichage selon un mode commandé réversible de l'affichage des autres interfaces, en commandant sur toutes les autres interfaces un affichage identique à l'affichage de la première interface ;
   - apposer au moins une marque graphique d'annotation sur l'image à traiter pour générer une image annotée ;
   - enregistrer et transmettre de chaque image annotée sur la console avec un identifiant représentatif de l'interface ayant transmis l'image annotée ;
- si l'affichage en mode commandé est activé par un utilisateur, l'unité centrale de la console et l'unité centrale de chaque interface peuvent être programmées pour mémoriser préalablement un affichage initial de chaque interface, pour commander sur toutes les autres interfaces un affichage identique à l'affichage de la première interface, puis pour réafficher l'affichage initial de chaque interface lorsque le mode commandé est désactivé ;
- l'unité centrale de la console peut être programmée pour combiner les images annotées envoyées par chaque interface et générer une image résultat avec un algorithme combinatoire analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes ;
- l'unité centrale de la console peut être programmée pour générer une image résultat dans laquelle des marques d'annotations graphiques des images annotées présentant un taux de similitude supérieur à un seuil de similitude déterminé sont affichées selon un premier format graphique, et dans laquelle des marques d'annotations graphiques des images annotées présentant un taux de similitude inférieur au seuil de similitude déterminé sont affichées selon au moins un deuxième format graphique ;
- l'unité centrale de la console peut être programmée pour exécuter une analyse statistique des marques d'annotations graphiques ;
- l'analyse statistique peut comprendre un calcul de pourcentage de marques d'annotations graphiques présentant un taux de similitude supérieur à un seuil de similitude déterminé et un calcul de pourcentage de marques d'annotations graphiques présentant un taux de similitude inférieur au seuil de similitude déterminé ;
- le seuil de similitude peut être paramétrable ;
- l'unité centrale de la console et/ou de chaque interface peut être également programmée pour générer une table de correspondance entre chaque marque d'annotation graphique, définie au moins par des coordonnées de position dans l'image annotée, et son auteur ;
- l'unité centrale de la console peut être également programmée afficher un identifiant d'auteur de chaque marque d'annotation graphique sur l'image résultat ;
- l'unité centrale de chaque interface peut être programmée pour être activable avec un code administrateur ou avec un code utilisateur, l'unité centrale de la console étant programmée pour afficher sur une interface activée avec le code administrateur, des outils d'administration comprenant : une liste d'images à traiter présentes dans la mémoire de l'unité centrale, une liste d'identifiants des participants, une liste pour des descripteurs d'objets ou de région d'intérêt sélectionnables par les utilisateurs, chaque descripteur étant associés à une marque d'annotation graphique prédéfinie différente ;
- la console peut comprendre une prise de connexion à une mémoire amovible reliée à l'unité centrale de la console ;
- la console peut comprendre une prise de connexion réseau reliée à l'unité centrale de la console, cette dernière étant programmée pour communiquer avec un serveur distant via un réseau de communication pour télécharger au moins une image à traiter et à la stocker en mémoire ;
- le serveur distant peut comprendre une première mémoire de stockage d'images originales associées chacune à des informations d'identification confidentielles, une deuxième mémoire de stockage d'images à traiter correspondantes, dépourvues des informations d'identification confidentielles mais associées chacune à un identifiant unique, et un tableau de correspondance entre les images originales et les images à traiter ;
- l'unité centrale de la console peut être programmée pour ajouter à l'image résultat obtenue après annotation d'une image à traiter l'identifiant unique de l'image à traiter et pour transmettre l'image résultat munie de l'identifiant unique au serveur distant ;
- l'unité centrale de la console et l'unité centrale des interfaces peuvent être programmées pour afficher un guide d'annotation séquentiel accompagné d'un référentiel terminologique, pour enregistrer séquentiellement les réponses de chaque expert, et pour générer un pré rapport d'examen comprenant un plan d'examen de l'image à traiter dans lequel les réponses de chaque expert sont reportées et combinées séquentiellement ;
- le pré rapport d'examen peut comprendre un identifiant unique de l'image à traiter, l'image résultat, et un compte-rendu normé d'une analyse statistique de l'image résultat ;
- le système peut comprendre au moins deux consoles de distribution d'images à traiter et de collecte d'images annotées aptes à communiquer entre elles, les consoles étant programmées pour avoir un statut paramétrable permettant une organisation hiérarchique des consoles ; et/ou
- l'une des consoles peut être programmée pour présenter un statut principal alors que les autres consoles présentent un statut secondaire, la console présentant un statut principal étant apte à commander les consoles présentant un statut secondaire;

On entend par « format graphique », le format visuel de représentation d'une annotation ; il peut s'agir essentiellement d'une couleur, d'une forme (carré, rond, croix, etc.), d'un trait de contour (pointillé, trait plein, trait de triangles, trait de carrés, etc.). Le format graphique ne doit pas être confondu avec le « format numérique » qui est le format d'un fichier interprétable par un ordinateur : par exemple le format numérique texte (txt, doc, etc.), et le format numérique image (bpm, jpeg, tiff, etc.).

L'invention a également pour objet un procédé de traitement d'image collaboratif et participatif comprenant les étapes suivantes :
- placer une image à traiter dans une mémoire d'une console apte à communiquer localement avec au moins deux interfaces de traitement ;
- transmettre une copie de l'image à traiter à chaque interface de traitement depuis la console de distribution ;
- sur chaque interface de traitement, afficher des outils de modification graphique de la copie de l'image à traiter pour permettre à un utilisateur de modifier graphiquement la copie de l'image à traiter et ainsi générer une image annotée ;
- transmettre chaque image annotée sur la console de distribution depuis chaque interface de traitement, et
- générer avec la console de distribution une image résultat à partir des images annotées à l'aide d'un algorithme combinatoire déterminé analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes.

Selon d'autres modes de réalisation, qui peuvent être combinés entre eux :
- les annotations graphiques des images annotées présentant un taux de similitude supérieur à un seuil de similitude déterminé peuvent être marquées selon un premier format graphique dans l'image résultat, et dans lequel les annotations graphiques de différentes images présentant un taux de similitude inférieur au seuil de similitude déterminé peuvent être marquées selon au moins un deuxième format graphique dans l'image résultat. Le procédé comprend, en outre, les étapes suivantes :
   - permettre un affichage selon un mode individuel dans lequel l'affichage de chaque interface est indépendant des autres interfaces, et
   - permettre un affichage selon un mode commandé dans lequel l'affichage d'une première interface commande l'affichage sur les autres interfaces, le passage entre le mode individuel et le mode commandé étant réversible.

D'autres caractéristiques de l'invention seront énoncées dans la description détaillée ci-après, faite en référence aux dessins annexés, qui représentent, respectivement :
la figure 1, une vue schématique d'un premier mode de réalisation d'un système collaboratif et interactif de traitement d'image selon l'invention dans lequel toutes les images affichées sont identiques ;
la figure 2, une vue schématique d'un système collaboratif et interactif de traitement d'image selon l'invention dans lequel les images affichées sont différentes d'une interface à une autre ;
la figure 3, une vue schématique en plan vue de dessus d'un exemple d'affichage sur une interface de traitement selon l'invention ;
les figures 4 et 5, des vues schématiques en plan vues de dessus respectivement d'une image sur laquelle un objet d'intérêt a été annoté par deux utilisateurs différents, et d'une image affichant la différence d'annotations entre les deux utilisateurs en vue d'une analyse statistique ;
la figure 5a, une vue schématique en plan vue de dessus d'une image affichant la différence d'annotations entre plusieurs groupes d'utilisateurs en vue d'une analyse statistique ; et
les figures 6 à 9, des vues schématiques en plan vue de dessus respectivement d'une image à traiter, d'une image annotée par un premier utilisateur, d'une image annotée par un deuxième utilisateur et une image résultat générée par la console.

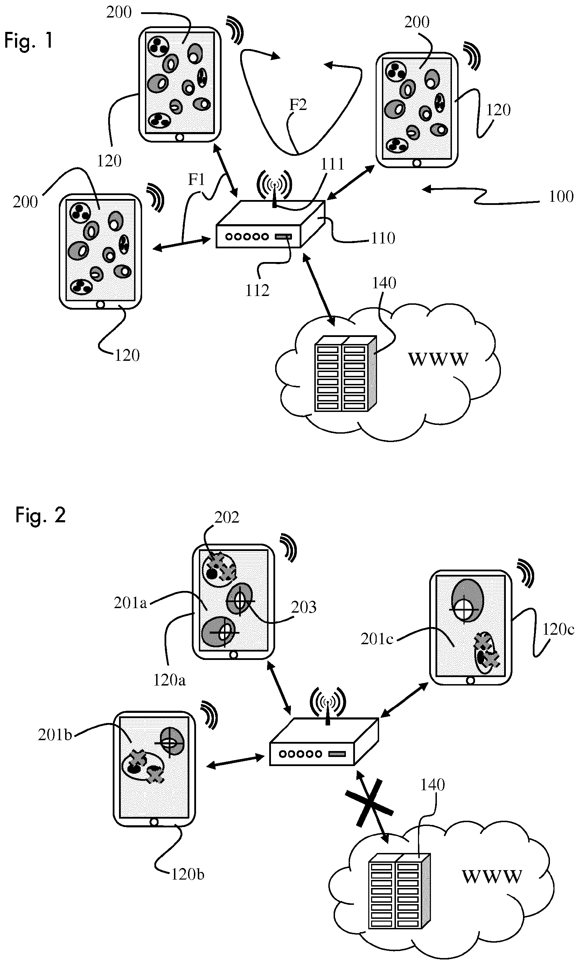

Un exemple de réalisation d'un système de traitement d'image selon l'invention est illustré en figure 1.

Le système 100 comprend une console 110 de distribution d'images à traiter et de collecte d'images annotées, et au moins deux interfaces de traitement 120 aptes à afficher une image à traiter 200.

La console 110 est apte à communiquer localement avec les interfaces 120, comme illustré par les flèches F1.

Plus précisément, la console 110 comprend un émetteur/récepteur sans fil 111 d'un signal sans-fil local, de type wifi avantageusement, LoRa ou SigFox. Elle comprend également une mémoire de stockage (non illustrée) pour au moins une image à traiter, pour au moins une image résultat et de préférence pour les images annotées.

Elle comprend, en outre, une unité centrale (non illustrée) programmée pour mettre en œuvre le procédé de traitement d'image selon l'invention.

De préférence, la console comprend également une prise de connexion 112 pour une mémoire amovible, la prise étant reliée à l'unité centrale de la console. Cela permet par exemple de télécharger des images depuis une prise USB.

La console 110 peut également comprendre une connexion réseau (soit sans fil soit filaire par l'intermédiaire d'une prise de connexion Internet reliée à l'unité centrale de la console) pour être reliée par un réseau de communication, tel qu'Internet, à un serveur distant 140. L'unité centrale de la console 110 est alors programmée pour communiquer avec le serveur distant 140 pour télécharger au moins une image à traiter et pour la stocker en mémoire.

Il peut s'agir, de préférence, d'un mini-routeur NAS (de l'anglais Network Attached Storage) sans ventilateur, émettant un signal Wifi, LoRa ou SigFox, local et stable. Ce type de console est portable, donc facilement déplaçable, et elle fait peu de bruit, de sorte que la séance de travail n'est pas gênée.

De préférence, lorsque la console 110 comprend une connexion à Internet, l'unité centrale de la console 110 est programmée pour rompre cette connexion lors du traitement d'image, et pour rétablir cette connexion uniquement lors du transfert sécurisé de l'image résultat et/ou d'un rapport d'examen au serveur distant 140. Grâce à cela, la connexion se fait à l'avance, lors de la préparation de la séance de travail, et s'interrompt ensuite, de sorte que le travail en groupe n'est pas dépendant de la qualité de la communication avec le serveur.

Les interfaces de traitement 120, ou terminaux de traitement, sont avantageusement des tablettes tactiles, de préférence équipées de stylets pour permettre une annotation des images directement sur l'écran pour plus de précision.

Alternativement, il peut s'agir d'ordinateurs portables. Dans ce cas, l'annotation des images se fait par l'intermédiaire d'une souris ou d'un pavé tactile. Ce qui importe c'est que l'utilisateur puisse manipuler les images (déplacement, agrandissement/rétrécissement, les annoter), et communiquer avec la console 110.

À cette fin, les interfaces 120 comprennent un émetteur/récepteur sans fil (non illustré) d'un signal sans-fil local compatible avec l'émetteur/récepteur 111 de la console 110 afin de permettre un échange local de données via le signal sans fil (flèches F1). Chaque interface 120 comprend également une mémoire de stockage pour au moins une image à traiter et pour au moins une image annotée, et une unité centrale.

La communication locale est stable et de bonne qualité. En outre elle peut être cryptée et permettre une sécurisation du travail puisque celui-ci se fait « hors-ligne », sans risque que les résultats soient piratés.

### PREPARATION DE LA SEANCE DE TRAVAIL

Une séance de travail est préparée et animée par un utilisateur dit « administrateur ». Il peut s'agir de l'un des experts qui va participer à la séance de travail ou non.

Dans le système selon l'invention, l'unité centrale de chaque interface est programmée pour être activable avec un code administrateur ou avec un code utilisateur.

Avant une séance de travail, la personne en charge de la séance de travail entre un code administrateur sur l'une des interfaces, ce qui a pour effet de connecter l'interface à la console et permet d'accéder aux outils d'administrations de la console. L'unité centrale de la console est programmée pour afficher sur l'interface activée avec le code administrateur, les outils d'administration comprenant : une liste d'images à traiter présentes dans la mémoire de l'unité centrale (ou sur une mémoire externe branchée à la console ou sur une mémoire d'un serveur distant), une liste pour le nom des participants, une liste pour des descripteurs d'objets et/ou de régions d'intérêt sélectionnables par les utilisateurs associés chacun à une marque d'annotation prédéfinie différente (des formes différentes, des formats de traits différents, des couleurs différentes, etc.), une liste de types d'examen préenregistré ou un module d'enregistrement d'un nouveau type d'examen préparé par l'administrateur.

Concernant les images, la ou les lames virtuelles (ou autres types d'images) dans leur format propriétaire d'origine (chaque entreprise de scan de lame dispose de son propre format d'image) sont acheminées par l'administrateur vers la console de distribution, soit par une clé USB, soit grâce à une connexion à distance avec le serveur de stockage distant 140.

La console de distribution 110 convertit alors automatiquement les images dans un format standard, avantageusement le format JPEG.

Elle génère ainsi une banque d'images, stockée localement dans la mémoire de la console 110, et une liste de ces images afin de permettre, par la suite, de sélectionner les images et de les traiter sur les interfaces 120.

Dans un premier mode de réalisation, chaque image reste entière et ne comprend donc qu'une seule image à traiter dont une copie sera envoyée sur chaque interface pour le traitement. Ce mode de réalisation est réservé, de préférence, à des images peu encombrantes pour limiter le temps de transfert entre la console 110 et les interfaces 120.

L'invention propose également un mode de réalisation avantageux pour les images très lourdes, comprenant un grand nombre de pixels, afin de réduire le temps de transfert et améliorer la fluidité.

Dans ce mode de réalisation, seule une copie d'une portion de chaque image de base est envoyée sur chaque interface.

A cette fin, la console de distribution 110 découpe chaque image de base en une série de tuiles (par exemple de taille 256 pixels x 256 pixels ou 512 pixels x 512 pixels). La console 110 associe à chaque image (et donc à chaque tuile) une grille de cartographie afin de générer un couple de coordonnées (x,y) unique pour chaque pixel dans l'image afin de connaître la place de chaque tuile dans l'image initiale. Les détails du processus seront décrits par la suite.

Cela permet de réduire le poids des données échangées entre la console 110 et les interfaces 120 lorsqu'un utilisateur zoome sur une partie de l'image, ce qui fluidifie au maximum la manipulation des images (déplacements, zoom, etc.).

Puis, l'administrateur renseigne également la liste des participants pour que chaque image annotée lors de la séance de travail puisse être associée à l'expert l'ayant annotée.

Ensuite, l'administrateur prépare la liste des objets d'intérêt et/ou régions d'intérêt qui devront être identifiés par les participants. Soit les descripteurs font partie d'une liste préétablie, soit l'administrateur dispose d'un outil d'édition pour créer de nouveaux descripteurs. Puis il associe à chaque objet d'intérêt et/région d'intérêt une marque d'annotation.

Dans un mode de réalisation préféré, l'étape précédente se fait automatiquement, car l'administrateur peut associer au préalable un type d'examen prédéfini à chaque image.

Chaque type d'examen est associé à une liste des OOI et/ou ROI et leurs marques d'annotation correspondantes.

Grâce à cela, pour chaque image associée à un type d'examen, les utilisateurs auront des outils d'annotation graphique prédéfinis. Ainsi, pour un examen donné, ils devront délimiter graphiquement des zones d'intérêt et/ou ajouter une marque graphique sur des objets d'intérêt prédéfinis, comme par exemple la présence d'infiltrat inflammatoire par la visualisation de lymphocytes ou de zones hémorragiques par la présence d'hématies, etc. À chaque descripteur est associé une marque d'annotation graphique prédéfinie (par exemple une surface libre, une croix, un carré, un rond, un triangle, en traits pleins ou pointillés, des couleurs différentes, etc.).

Avantageusement, chaque type d'examen est également associé à :
- un guide d'annotation séquentiel ; et, de préférence
- un référentiel terminologique.

Un guide d'annotation séquentiel est un ensemble de questions et/ou d'indications et/ou d'instructions qui guident séquentiellement, c'est-à-dire étape par étape dans un ordre donné, chaque expert pour mener son examen à bien. La séquence des questions et/ou d'indications et/ou d'instructions peut avantageusement être déterminée par un arbre de décision préstructuré. En d'autres termes, une question/indication/instruction peut dépendre de l'action prise par l'expert en réponse à une question/indication/instruction précédente.

Ainsi, pour chaque image associée à un type d'examen, les utilisateurs devront répondre à un certain nombre de questions et/ou d'indications et/ou d'instructions affichées de manière séquentielle et dynamique (c'est-à-dire en fonction de leur réponse à la question précédente). Par exemple, pour un examen donné, les experts devront répondre à une question sur la régularité et/ou l'étendue des contours des cellules observées, sur leur regroupement ou leur dispersion, etc.

Le système prévoit également que les experts pourront avoir à leur disposition un référentiel terminologique (par exemple en référence à une ontologie spécifique dans le domaine médical). Il s'agit d'une base de données contenant des entrées terminologiques et des informations associées. C'est un dictionnaire qui assure que les experts utilisent pour leurs commentaires, les mêmes termes pour les mêmes notions à des grossissements identiques.

Ainsi, lors de l'examen, l'unité centrale de la console et des interfaces sont programmées pour afficher, avec la copie de l'image, le guide d'annotation séquentiel accompagné du référentiel terminologique, pour enregistrer séquentiellement les réponses de chaque expert, et pour générer un pré rapport d'examen comprenant un plan d'examen de l'image à traiter dans lequel les réponses de chaque expert sont reportées et combinées séquentiellement.

Outre l'intérêt d'éviter d'oublier des étapes d'examen et d'employer un langage uniforme en guidant les experts dans leur examen lors de la séance de travail, l'utilisation d'un guide d'annotation séquentiel et un référentiel terminologique permet de générer un pré rapport complet à partir de toutes les données générées par chaque expert, sans en mettre de côté parce que n'étant pas prévue. À ce titre, une rubrique de type « remarques générales » prévue dans le guide d'annotation peut permettre à un expert de faire une observation non prévue dans la séquence d'examen et qui pourrait être pertinente, tout en étant prise en compte dans le prérapport.

Avantageusement, les types d'examens sont préenregistrés et disponibles soit dans la mémoire de la console 110, soit en ligne dans une base de données dédiée.

Ainsi, les guides d'annotation séquentielles et les référentiels terminologiques peuvent être préparés et conçus par des autorités professionnelles et assurer ainsi une uniformisation des examens.

Alternativement, le type d'examen peut être préparé par l'administrateur lui-même. Dans ce cas, il définira lui-même :
- De manière optionnelle : un guide d'annotation séquentiel et un référentiel terminologique
- Les descripteurs (c'est-à-dire les OOI et/ou les ROI) qu'il souhaite mettre à disposition des experts, et il les associera à une marque d'annotation graphique donnée (par exemple une couleur de zone de forme libre à délimiter, une croix, un carré, un rond, un triangle, en traits pleins ou pointillés, etc.).

Dans les deux cas (examen préétabli ou examen préparé par l'administrateur), le système est capable de dénombrer les marques de chaque OOI ou ROI en mémorisant le nombre de marques d'annotation disposées par chaque expert.

En outre, le système selon l'invention est également programmé pour associer à chaque marque d'annotation au moins un couple de coordonnées x, y dans l'image ainsi qu'une identification de l'expert qui l'a déposée. Ainsi, l'unité centrale de la console et/ou de chaque interface est/sont programmée(s) pour générer une table de correspondance entre chaque marque, définie par ses coordonnées de position dans l'image annotée, son type (croix, carré, rond, triangle, traits pleins ou pointillés, couleur de zone libre à délimiter, etc.), éventuellement son étendue (en nombre de pixels), l'OOI ou la ROI qu'elle représente, et son auteur.

Une fois la séance préparée, l'administrateur peut mettre fin à sa session administrateur et, s'il participe à l'examen, se connecter avec un code utilisateur classique.

Si les images ont été téléchargées depuis un serveur distant, l'unité centrale de la console 110 est avantageusement programmée pour rompre la connexion réseau avant l'examen (comme illustré en figure 2), ce qui permet de s'assurer, d'une part qu'il n'y ait aucune connexion non autorisée lors de l'examen, et d'autre part une parfaite fluidité du travail, indépendamment de la qualité d'une connexion réseau.

### DEROULEMENT DE LA SEANCE DE TRAVAIL

Seuls les utilisateurs prévus dans la liste créée par l'administrateur peuvent accéder à la banque d'images, à l'aide au moins d'un identifiant et d'un mot de passe. D'autres moyens de sécurisation d'accès peuvent être utilisés, tels que l'identification rétinienne ou d'empreintes digitales.

En outre, les communications entre la console et les interfaces sont de préférences cryptées.

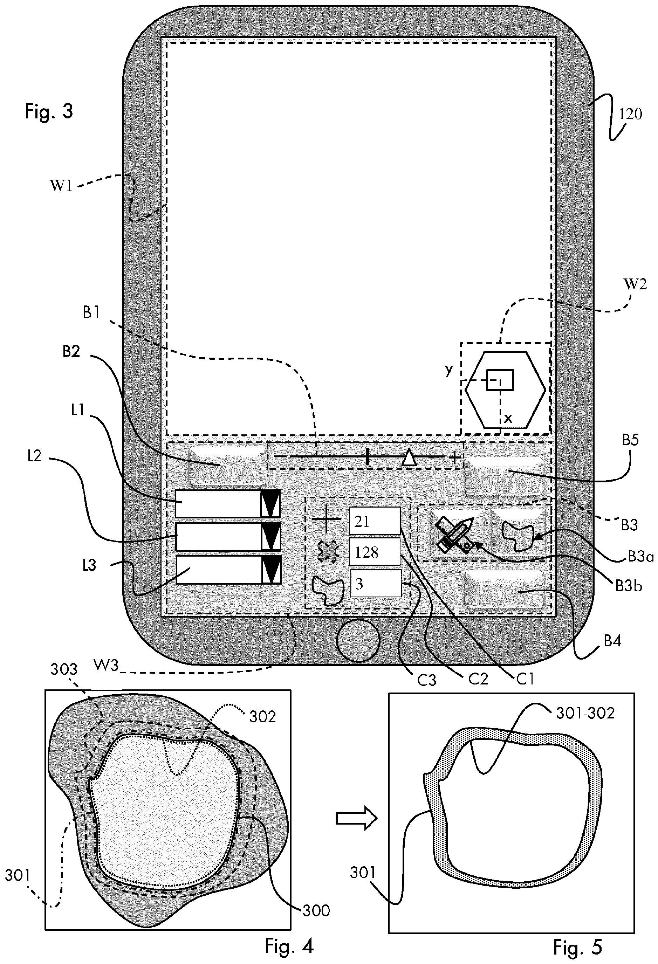

Un exemple d'affichage sur une interface 120 est illustré en figure 3.

Une première zone W1 est réservée à l'affichage de l'image proprement dite. Une zone W2, plus petite que W1, représente l'image entière de l'échantillon analysé (schématisé par un hexagone sur la figure 3), ainsi que la partie de l'image affichée dans la zone W1 avec sa localisation en données x, y dans l'image entière. L'utilisateur est ainsi capable de savoir à tout moment où se situe la portion qu'il visualise par rapport au reste de l'échantillon.

Une troisième zone W3 regroupe différentes données et boutons d'interaction nécessaire à l'examen.

Par exemple, la zone W3 comprend un indicateur de zoom B1 de l'image affichée par rapport à l'image entière.

La zone W3 comprend, en outre, un bouton de connexion B2 permettant soit de mettre fin à la session, soit de passer en mode administrateur pour ajouter, par exemple, un descripteur à mettre à disposition des experts pour l'examen.

La zone W3 comprend également une liste déroulante L1 des images à afficher et une liste déroulante L2 des régions d'intérêt à placer sur l'image et à examiner. Ces deux listes ont été sélectionnées par l'administrateur lors de la phase de préparation de la session de travail.

La zone W3 comprend aussi une zone d'outils d'annotation graphique B3, comprenant par exemple un bouton B3a d'activation d'une barre d'outils pour délimiter une région d'intérêt et un bouton B3b d'activation d'une barre d'outils de différentes marques graphiques d'annotation d'objets d'intérêt avec leur descripteur.

La zone d'affichage W3 comprend aussi des compteurs C1, C2 et C3 des annotations (marques et ROI). Dans l'exemple illustré, 21 croix droites (correspondant à un premier type d'OOI) ont été placées, 128 croix obliques (correspondant à un deuxième type d'OOI) ont été placées, et 3 régions d'intérêt ont été délimitées.

Enfin, la zone d'affichage W3 comprend un bouton B4 de sauvegarde du travail effectué. Alternativement ou en combinaison, l'unité centrale de chaque interface 120 peut être programmée pour sauvegarder automatiquement l'image annotée à intervalle régulier. La sauvegarde manuelle ou automatique se fait en local dans une mémoire de l'interface 120 et/ou à distance, dans une mémoire de la console 110.

Avantageusement, la zone W3 comprend également un menu déroulant L3 affichant des modules d'annotation spécifiques dédiés à des pathologies particulières.

### ANNOTATION

Selon l'invention, la console 110 et les interfaces 120 sont programmées pour permettre une transmission d'une copie 200 de l'image à traiter (l'image de base entière ou une tuile, c'est-à-dire une portion de l'image de base entière) sur chaque interface de traitement 120 depuis la console de distribution 110 pour permettre une modification graphique de la copie de l'image à traiter sur chaque interface 120 pour obtenir une image annotée 201a, 201b, 201c.

Une copie de l'image de base entière, de basse résolution et de petite taille (de type imagette, donc très légère) est transmise à chaque interface et affichée dans la zone d'affichage W2. Cette copie constitue un plan de l'image de base à traiter.

Lorsque l'image de base est légère, une copie de l'image de base entière est transmise à chaque interface 120 pour affichage dans la zone W1 et pour annotation et examen.

L'utilisateur peut ensuite zoomer dans l'image entière affichée en zone W1.

Lorsque l'image de base est lourde, elle est découpée en tuiles constituant une pluralité d'images à traiter. Dans ce cas, une copie d'une seule tuile à traiter, définie par défaut, est transmise avec le plan et affichée en entier dans la zone W1.

Soit l'utilisateur annote cette tuile et peut s'y déplacer ou zoomer dedans, soit il cherche à se déplacer dans l'image entière. Dans ce cas, en fonction du zoom dans l'image et du déplacement opéré par l'utilisateur depuis son interface 120, cette dernière produit une requête qui est envoyée à la console 110. Elle comprend a minima : les coordonnées (x,y) de référence et un couple de dimensions en x et en y (en nombre de pixels). La console 110, suivant la requête, transmet la ou les tuiles correspondantes à l'interface qui la ou les affiche en entier dans la zone W1. L'utilisateur peut alors zoomer dans ces nouvelles tuiles, s'y déplacer et les annoter.

Chaque expert visualise une copie de l'image à traiter (l'image entière ou au moins une tuile) sur son interface 120 et il peut la manipuler (se déplacer dans l'image, l'agrandir ou la rétrécir) et l'annoter graphiquement (c'est-à-dire modifier les pixels qui la composent).

Ce travail, bien que réalisé en même temps par tous les experts, est également fait de manière indépendante par chaque expert. Ainsi, comme le montre la figure 2, la console et les interfaces sont programmées pour permettre un affichage selon un mode individuel dans lequel l'affichage de chaque interface est indépendant des autres interfaces : l'expert manipulant l'interface 120a annote une partie 201a de l'image, l'expert manipulant l'interface 120b annote une partie 201b de l'image, et l'expert manipulant l'interface 120c annote une partie 201c de l'image.

Les marques graphiques d'annotation prévues dans l'exemple illustré sont des croix obliques (X) 202 et des croix droites (+) 203. Ces marques remplacent les pixels de l'image sur laquelle elles sont positionnées.

La pose de marques d'annotations peut être faire suivant deux modes :
a) Un mode simple dans lequel l'administrateur a défini des catégories d'OOI et les utilisateurs ajoutent des marques d'annotation graphiques sur les éléments de l'image à partir de ces catégories. Il s'agit d'une opération de comptage d'OOI (et/ou ROI) et/ou de localisation des OOI (et/ou ROI) et/ou d'évaluation de l'étendue des OOI/ROI délimités par chaque expert.
b) Un mode guidé, dans lequel en plus des différentes catégories d'OOI et/ou ROI, l'administrateur définit ou choisit un guide d'annotation séquentiel comprenant des séquences d'annotations suivant la logique d'un arbre de décisions. L'unité centrale de la console et des interfaces sont programmées pour afficher avec la copie de l'image un guide d'annotation séquentiel accompagné d'un référentiel terminologique, pour enregistrer séquentiellement les réponses de chaque expert. Ainsi, chaque utilisateur répond à des questions fermées définies par le guide d'annotation séquentiel. En fonction de chaque réponse, la console ou l'interface (selon l'implémentation choisie du système selon l'invention) détermine quelles marques d'OOI/ROI afficher dans les barres d'outils B3a et B3b, en fonction de la séquence correspondant.
   Puis l'utilisateur pose ses marques d'annotations graphiques pendant chaque séquence, ce qui modifie les pixels de l'image, et enchaîne avec la question suivante. Toutes les catégories ne sont donc pas accessibles simultanément. L'utilisateur est ainsi guidé dans son diagnostic.

Grâce à ce mode de fonctionnement, la terminologie et les séquences d'examen peuvent être produites et validées par la profession (des experts du domaine) avant tout examen. Cela assure une homogénéisation des étapes de diagnostics, une minimisation des écarts de mode diagnostics entre groupes de praticiens (le compte-rendu final pourra comparer les écarts de diagnostics entre des utilisateurs qui ont suivi une méthodologie homogène et comparable sur des images identiques).

La production d'annotations depuis chaque interface (marques d'annotation pour les ROI et/ou OOI, le chemin suivi dans l'arbre de décision, la prise de copie d'écran et l'horodatage de toutes les données) peut être faite librement ou de manière imposée ; en imposant par exemple à chaque utilisateur, une zone spécifique à marquer. Chaque ROI dessinée par les différents experts est identifiée par son auteur, un numéro unique et l'image dans laquelle elle se trouve pour assurer une parfaite traçabilité. Les annotations sont ensuite envoyées à la console, c'est-à-dire que la position (x ;y) de chaque pixel de l'annotation est envoyée à la console. La production d'annotations en mode imposé peut être réalisée en présentiel de manière synchrone (simultanément par tous les utilisateurs) ou asynchrone (par tous les utilisateurs mais à des moments différents pendant la séance de travail).

L'horodatage facilite la traçabilité des actions de tous les utilisateurs, avec leur évolution dans le temps (éventuellement possibilité de produire des annotations en plusieurs fois). C'est utile pour consolider les résultats et comparer les évolutions (par exemple si même l'image est soumise aux mêmes experts, à deux moments différents)

Les annotations de chaque interface sont comptabilisées selon le type et leur place est enregistrée à l'aide d'au moins un couple de coordonnées x, y dans l'image.

Puis l'image annotée est enregistrée et transmise à la console 110.

Avantageusement, l'unité centrale de chaque interface est programmée pour générer une table de correspondance entre chaque marque d'annotation, définie au moins par des coordonnées de position dans l'image annotée, et son auteur. De préférence, le type de l'annotation et la séquence d'annotation servent également à définir chaque marque d'annotation. Ainsi, lorsque l'image annotée est enregistrée, ce n'est en fait que cette table de correspondance, représentative de l'image annotée, qui est transmise à la console 110. Cette dernière pourra ensuite replacer les pixels de chaque marque d'annotation sur l'image résultat qui sera générée, ce qui limite le volume de données transmis et donc contribue à la fluidité.

Ainsi, par « image annotée » dans la présente invention, on entend soit l'image annotée en tant que telle (par exemple au format jpeg) avec ses marques d'annotation graphiques, soit une table de données d'annotations graphiques représentative de l'image annotée.

### PARTAGE D'AFFICHAGE

Grâce au système selon l'invention, il est possible de permettre une véritable coopération entre les experts pendant l'examen.

À cette fin, la console et les interfaces sont programmées pour permettre un affichage selon un mode commandé dans lequel l'affichage d'une première interface commande l'affichage sur les autres interfaces, le passage entre le mode individuel et le mode commandé étant réversible.

L'affichage commandé selon l'invention permet à plusieurs utilisateurs de communiquer entre eux via la console, comme schématisé par la flèche F2 sur la figure 1.

Ainsi, un expert souhaitant attirer l'attention des autres experts ou souhaitant poser une question, est en mesure de montrer aux autres experts, sur leur propre interface, une zone spécifique de l'image.

Pour activer/désactiver le mode commandé, l'interface comprend un bouton B5 sélectionnable directement par un utilisateur sur l'écran de l'interface 120.

Seul l'affichage est commandé, les autres fonctionnalités du système restant disponibles sur chaque interface. Ainsi, au cours d'un affichage commandé par une première interface, il est possible aux utilisateurs des autres interfaces de profiter de l'occasion pour annoter graphiquement la portion de l'image qui est affichée.

Cette fonctionnalité d'affichage commandé réversible a un double intérêt :
Le premier est que lors de l'affichage commandé, seuls les coordonnées et le niveau de zoom sont envoyés aux autres interfaces, et non pas la portion d'image en tant que telle, ce qui assure une parfaite réactivité de l'affichage commandé et une parfaite fluidité.

En outre, chaque utilisateur reste maître de l'annotation de son image. En d'autres termes, même si la même portion d'image est affichée sur chaque interface, l'image elle-même reste spécifique de l'interface et donc de l'utilisateur et peut donc être annotée indépendamment des autres interfaces.

L'utilisateur ayant activé l'affichage commandé ne peut qu'attirer l'attention des autres utilisateurs sur une zone particulière de l'image, sans pour autant commander ou forcer l'annotation de leur propre image. Il reste alors possible d'analyser les résultats de chaque utilisateur sans biais statistique même si la fonction d'affichage commandée a été activée lors de l'examen collégial de l'image.

Selon un mode de réalisation avantageux, si l'affichage en mode commandé est activé par un utilisateur, l'unité centrale de la console et/ou l'unité centrale de chaque interface sont programmées pour mémoriser préalablement un affichage initial de chaque interface et pour commander sur toutes les interfaces un affichage identique à l'affichage de la première interface.

L'affichage initial est avantageusement constitué par une valeur de zoom représentative de l'agrandissement de l'image affichée par rapport à l'image entière (c'est-à-dire une échelle en valeur réelle, interactive avec le grossissement) ainsi qu'une position de l'image affichée par rapport à l'image entière sous forme d'un couple de coordonnées x, y.

Ainsi, lorsqu'un premier utilisateur prend la main en activant le mode commandé, les autres utilisateurs n'ont pas à craindre de perdre la position ni le niveau d'agrandissement de l'image qu'ils étaient en train de visualiser au moment du passage en mode commandé.

Lorsque le mode commandé est désactivé, l'unité centrale de la console et/ou l'unité centrale de chaque interface sont programmées pour réafficher l'affichage initial de chaque interface. Chaque utilisateur se retrouve alors face à l'affichage dans lequel il était avant l'activation du mode commandé et peu continuer son travail.

Ce réaffichage de la position initiale peut être automatique ou sur requête de chaque utilisateur afin de leur laisser le temps d'annoter la portion d'image affichée avant de revenir à la partie qu'ils étaient en train d'annoter avant l'affichage commandé.

Ce mode de fonctionnement permet simultanément un partage d'image et de points de vue. L'utilisateur qui a pris la main peut montrer directement aux autres la zone d'image qui lui pose un problème ou sur laquelle il souhaite attirer leur attention.

En outre, la petite quantité d'information transmise (coordonnées x, y des pixels des annotations et valeur de zoom) permet une navigation et un affichage très fluide et rapide.

Une fois le travail terminé et l'image entièrement annotée graphiquement et éventuellement commentée conformément au guide d'annotation séquentiel et au référentiel terminologique, la console 110 et les interfaces 120 sont également programmées pour permettre une transmission (automatique ou manuelle sur instruction des utilisateurs) de chaque image annotée sur la console de distribution 110 depuis chaque interface de traitement 120. Comme décrit précédemment, il peut s'agir de l'image annotée proprement dite, mais il s'agit de préférence, d'une table de données d'annotations représentatives de l'image annotée, et comprenant les correspondances entre chaque marque d'annotation, définie par le type de l'annotation (selon son descripteur, par exemple) et des coordonnées de position dans l'image annotée, et son auteur. Une telle table comprend beaucoup moins de données qu'une image, de sorte que le transfert se fait très rapidement, et plusieurs experts peuvent transférer leurs annotations en même temps.

### GENERATION DE L'IMAGE RESULTAT

Après réception des données d'annotation (images annotées ou tables de données d'annotation représentatives de chaque image annotée), l'unité centrale de la console 110 est également programmée pour générer une image résultat à partir des images annotées avec un algorithme combinatoire déterminé permettant d'identifier (c'est-à-dire d'illustrer) les points communs et les différences entre les images annotées.

D'une manière générale, l'algorithme combinatoire analyse les modifications graphiques de chaque image annotée pour déterminer les modifications graphiques similaires et les modifications graphiques différentes entre les images annotées. Puis, il synthétise cette analyse et génère une image résultat constituée de pixels de formats graphiques différents entre les modifications graphiques similaires et les modifications graphiques différentes.

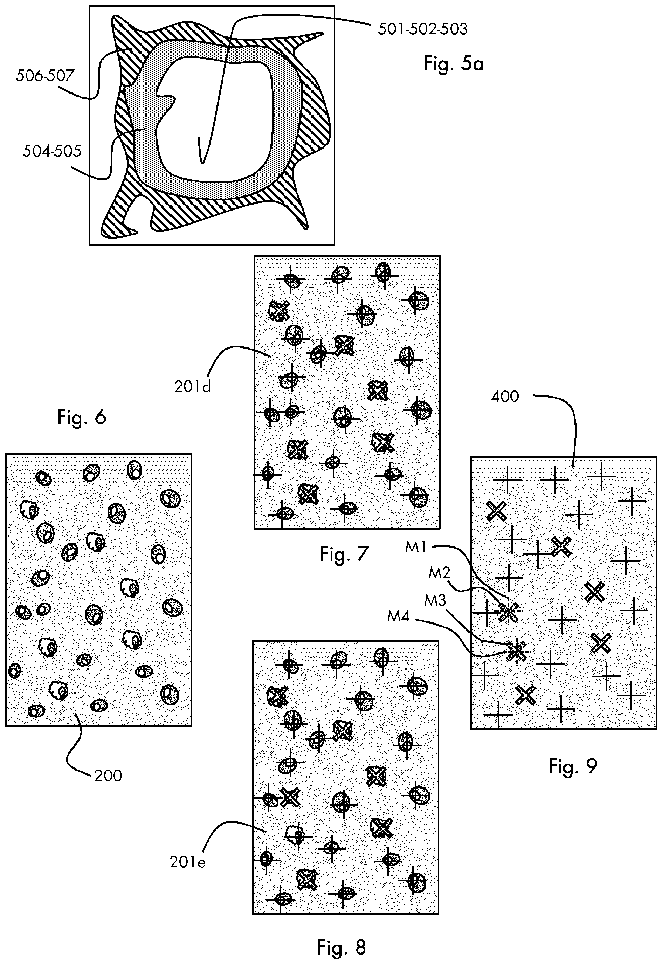

Un exemple simplifié est illustré aux figures 4 et 5.

Sur cet exemple, une région d'intérêt 300 est illustrée en ligne pleine. Un premier utilisateur a délimité cette région d'intérêt à l'aide d'une forme représentative 301 sur son interface, illustrée en ligne pointillée, un deuxième utilisateur a délimité cette région d'intérêt à l'aide d'une forme 302 sur son interface, illustrée en ligne points-tirets et un troisième utilisateur a délimité cette région d'intérêt à l'aide d'une forme 303 sur son interface, illustrée en ligne tiretée.

L'unité centrale de la console est programmée pour générer une image résultat, de préférence de format identique à l'image à traiter (c'est-à-dire de même format que les images annotées), dans laquelle les marques d'annotations (c'est-à-dire les pixels des marques d'annotation) des images annotées présentant un taux de similitude (par exemple en forme, en position, en type de marque) supérieur à un seuil de similitude déterminé sont fusionnées et affichées selon un premier format graphique (lignes, couleurs, format de trait, emplacement), et dans laquelle les marques d'annotations de différentes images présentant un taux de similitude inférieur au seuil de similitude déterminé sont fusionnées et affichées selon au moins un deuxième format graphique.

Dans l'exemple, illustré en figure 5, les régions d'intérêt 301 et 302 des premier et deuxième experts ont été fusionnées et regroupées sous forme d'une seule première ligne 301-302, car elles présentent un taux de similitude très proche : les experts ont bien délimité la zone d'intérêt 300. Même s'il existe une petite différence entre les deux, elle n'est pas de nature à changer l'examen. Les ROI 301 et 302 présentent un taux de similitude supérieur à un seuil de similitude déterminé.

Le seuil de similitude peut être déterminé soit par l'administrateur, soit pré-réglé dans le système. Alternativement ou en combinaison, le seuil de similitude peut être réglable, avant ou après la génération de l'image résultat, par exemple lorsque cette dernière comprend trop de marques différentes en raison d'un seuil de similitude trop élevé ne permettant pas de regrouper suffisamment les marques des différents experts.

La ROI 303 délimitée par le troisième expert présente un taux de similitude avec les deux autres ROI 301 et 302 inférieur au seuil de similitude déterminé. Elle est donc affichée sous forme d'une deuxième ligne distante de la première.

La ligne représentant des ROI similaires peut être calculée et placée, par exemple, à la position moyenne des positions des lignes similaires. Alternativement, elle peut être choisie comme étant l'une des lignes similaires. Il en va de même pour la ligne des ROI non similaires. Alternativement, chaque ligne non similaire peut être affichée à la position à laquelle l'utilisateur l'a créé.

L'algorithme combinatoire au sens de l'invention consiste donc à fusionner les annotations graphiques par groupe de similitudes (c'est-à-dire les annotations graphiques identiques ou dont la différence est inférieure au seuil de similitude déterminé) et à les insérer dans l'image résultat sous forme de pixels de formats graphiques différents entre chaque groupe mais de même format graphique dans un même groupe. Par format graphique, on comprend une couleur, une combinaison de forme (par exemple une ligne de tirets, de point, de triangles, une ligne pleine, etc.

En d'autres termes, pour générer l'image résultat, l'algorithme part de l'image à traiter, analyse la similitude des annotations graphiques des différents experts à partir de leur image annotée et forme des groupes de similitude, puis synthétise son analyse sous la forme d'une image résultat dans laquelle les pixels originaux de l'image sont remplacés automatiquement par des pixels de formats graphiques spécifiques, directement identifiables par un observateur de l'image résultat. Par exemple, pour sélectionner automatiquement un format graphique identifiable directement par un observateur, l'algorithme peut comprendre une étape d'analyse du spectre des pixels de l'image ou de la zone de l'image dans laquelle le résultat de la combinaison doit être affiché, et une étape de sélection d'une couleur présentant un taux de contraste supérieur à un taux de contraste seuil par rapport aux pixels de l'image ou de la portion d'image analysée.

Concrètement, l'algorithme compare les pixels entre deux marques d'annotation et les classe en deux catégories : ceux qui sont identiques (c'est-à-dire que le même pixel est modifié les experts) et ceux qui sont différents (c'est-à-dire que les experts ne l'ont pas modifié de la même manière).

Puis l'algorithme détermine si les pixels différents sont éloignés des pixels identiques d'un nombre de pixels seuil déterminant le seuil de similitude. On peut, pour cela, utiliser une fenêtre de n pixels de côté qui balaye le pourtour de la zone des pixels identiques et modifier le classement des pixels différents s'ils sont dans la fenêtre (ils passent alors dans la catégorie des pixels identiques). Les pixels qui restent en dehors de la fenêtre restent considérés comme différents.

De préférence, on ajoute une étape de classement consistant à calculer le pourcentage de pixels différents restant par rapport au nombre de pixels identiques. Si ce pourcentage est inférieur à un pourcentage seuil, les pixels différents sont classés comme pixels identiques et les marques des deux utilisateurs sont considérées comme identiques, même si elles ne sont pas placées strictement au même endroit sur l'image. Si ce pourcentage est supérieur au pourcentage seuil, les pixels identiques sont affichés selon un premier format graphique et les pixels différents sont affichés selon un autre format graphique.

Cela vaut pour les marques de plus de deux utilisateurs bien entendu. Dans ce cas, les pixels similaires sont fusionnés par groupes de similitude et affichés selon autant de formats graphiques qu'il y aura de groupes de pixels.

Alternativement ou en combinaison, l'analyse peut être réitérée pour les pixels classés différents en première phase, afin de déterminer au moins deux groupes classés par similitude au sein de ces pixels. Cela aboutit ainsi à nuancer les différences, c'est-à-dire faire apparaître graphiquement plusieurs groupes de différences (voir le commentaire de la figure 5a).

Ainsi, les annotations venant de différents experts sont combinées graphiquement dans une image unique et non simplement juxtaposées, ce qui nuirait à l'évaluation de l'analyse de l'image, en particulier lorsque le nombre d'objets ou de régions d'intérêt et/ou d'experts est élevé.

L'image résultat unique générée par le système donne donc graphiquement et directement à l'utilisateur qui n'a pas besoin d'interpréter les images annotées au moins trois types informations :
- il existe ou non des différences d'analyse entre les experts ;
- quels experts ne sont pas d'accord (l'utilisateur n'a pas à déterminer lui-même si les experts sont d'accord ou non dans leur analyse graphique de l'image) ;
- une information spatiale des différences et des points communs : leur localisation, leur forme.

Dans un mode de réalisation particulier, l'algorithme peut définir plusieurs groupes de différences. On peut ainsi nuancer les différences, c'est-à-dire faire apparaître plusieurs groupes d'annotations graphiques : les pixels communs, une première couronne de pixels avec un deuxième taux de similitude, une seconde couronne de pixels avec un troisième taux de similitude, etc. Cela permet de faire apparaître graphiquement la dispersion des avis des experts ou une hiérarchie des désaccords des experts : par exemple : similaires, presque similaires, différents, très différents.

Un exemple d'application, illustré à la figure 5a, concerne la délimitation préopératoire de l'étendue d'une tumeur.

Après analyse et synthèse par l'algorithme combinatoire, l'image résultat générée fait apparaître que les utilisateurs 501, 502 et 503 ont délimité la tumeur selon une première zone centrale de faible étendue. Les utilisateurs 504 et 505 ont considéré, eux, que la tumeur s'étendait un peu plus largement. Enfin, les utilisateurs 506 et 507 ont considéré que la tumeur s'étend encore plus largement.

Le système selon l'invention attire donc immédiatement l'attention sur le fait que les experts ne sont pas d'accord dans leur analyse. L'utilisateur déduit également directement de l'image résultat que certains utilisateurs (506-507) ont défini une tumeur dont la forme est très complexe et qui peut donc radicalement changer le traitement futur. Le système génère donc des données que l'utilisateur final n'aurait pas pu ou aurait très difficilement pu déduire de son analyse des images modifiées, surtout si le nombre de régions d'intérêt et/ou le nombre d'experts sont importants.

Le système permet donc à la fois de gagner un temps précieux, de confronter graphiquement et non sémantiquement, les analyses graphiques de différents experts, et d'améliorer la pertinence de leur diagnostique futur et du traitement choisi.

Les figures 6 à 9 illustrent un autre exemple d'obtention d'une image résultat concernant des objets d'intérêts marqués par des experts différents à l'aide du système selon l'invention.

La figure 6 représente l'image à traiter 200 envoyée à toutes les interfaces. La figure 7 représente l'image 201d annotée par un premier expert et la figure 8 illustre l'image 201e annotée par un deuxième expert. L'image 9, enfin, représente l'image résultat 400 générée par le système selon l'invention.

Dans l'image résultat, les marques d'annotations des images annotées par le premier et le deuxième expert présentant un taux de similitude (en position et en type de marque) supérieur à un seuil de similitude déterminé sont repérées selon un premier format graphique, ici en traits pleins. Les marques d'annotations M1, M2, M3 et M4 présentant un taux de similitude inférieur au seuil de similitude déterminé sont repérées selon au moins un deuxième format graphique, ici en traits pointillés.

De préférence, le procédé d'affichage selon l'invention comprend l'affichage de l'image résultat et l'affichage dynamique en surimpression du nom de l'auteur de chaque annotation lorsqu'un curseur de souris est déplacé sur l'image. Avantageusement, l'affichage dynamique ne concerne que les marques d'annotation M1, M2, M3 et M4 présentant un taux de similitude inférieur au seuil de similitude déterminé.

L'affichage dynamique est permis grâce à la table de correspondance, générée par le système selon l'invention (par l'unité centrale de la console et/ou de chaque interface), entre chaque marque d'annotation, définie au moins par ses coordonnées de position dans l'image annotée, et son auteur.

L'unité centrale de la console est également programmée pour exécuter une analyse statistique des marques d'annotations graphiques.

Par exemple, l'analyse statistique comprend le calcul d'un pourcentage de marques d'annotations présentant un taux de similitude supérieur à un seuil de similitude déterminé et le calcul d'un pourcentage de marques d'annotations présentant un taux de similitude inférieur au seuil de similitude déterminé.

Ce résultat permet d'indiquer si une image présente un fort consensus d'examen entre les experts ou non, et ce très rapidement et directement même s'il y a un grand nombre de marques et/ou un grand nombre d'experts. Cette analyse peut être plus fine et réalisée uniquement au sein des marques d'annotations divergentes. Il est alors possible de voir si une divergence est une simple erreur d'annotation : par exemple, pour un OOI donné (identifié par ses coordonnées x, y) et un panel d'expert de 20 personnes, si 95% des marques sont identiques et que seuls 5% sont différentes, alors il est probable qu'il s'agisse d'une erreur de marquage et l'on peut demander confirmation à son auteur. Au contraire, si un OOI a été annoté différemment par la moitié du panel, l'analyse statistique permet à l'expert responsable de l'examen, soit d'attirer l'attention sur cet OOI, soit de redemander au panel d'expert d'analyser cet OOI et d'en débattre. Le mode d'affichage commandé selon l'invention est, dans cette situation, particulièrement intéressant.

### GENERATION DU PRE-RAPPORT

Outre l'intérêt d'assurer qu'aucune phase de l'examen ne sera oubliée par un expert, l'utilisation d'un guide d'annotation séquentiel et d'un référentiel terminologique permet au système selon l'invention de générer automatiquement, après l'image résultat, un prérapport d'examen qui devra bien sûr être complété par l'expert responsable de l'examen.

Le contrôle qualité d'une structure dans l'industrie, ou un examen médical nécessitent un examen de différents points de contrôle. Idéalement, afin d'assurer la rigueur du contrôle, l'examen est organisé en catégories de contrôle, selon une séquence bien déterminée. Malheureusement, ça n'est pas toujours le cas, et le contrôleur ou le médecin remplit son compte-rendu au fur et à mesure de son examen. Indépendamment de la compétence du contrôleur, cela entraîne des difficultés dans la traçabilité et dans la reproductibilité du contrôle : en effet, certains points peuvent être omis. En outre, il peut y avoir une grande variation de descripteurs de l'image selon le vocabulaire de chacun, ce qui peut entraîner des risques d'incompréhension. Enfin, la comparaison de deux examens effectués par des personnes différentes à des moments différents peut être très fastidieuse voire impossible lorsque les plans de compte-rendu ne sont pas identiques.

Le système selon l'invention permet également de remédier à cela.

Une fois que la console a généré l'image résultat, elle génère également un pré rapport d'examen comprenant un plan d'examen de l'image à traiter dans lequel les réponses de chaque expert sont reportées et combinées séquentiellement. Le plan du prérapport et son contenu sont déterminés par le guide d'annotation séquentiel (plan d'examen et questionnaire) et le référentiel terminologique qui l'accompagne et qui ont été sélectionnés (ou créés) par l'administrateur lors de la préparation de la séance de travail.

Une fois que l'image résultat a été générée, l'unité centrale de la console est programmée pour générer, selon le plan d'examen de l'image à traiter, un pré rapport d'examen comprenant l'identifiant unique de l'image à traiter, l'image résultat et les réponses des experts au questionnaire.

De préférence, le prérapport comprend également un compte-rendu normé de l'analyse statistique de l'image résultat. En d'autres termes, le prérapport comprend les résultats de l'analyse statistique sous forme de phrases préétablies. Par exemple : «Dans la catégorie A (prédéfinie par l'administrateur) des Objets d'intérêt, X1 pourcents des experts ont trouvé moins de N1 occurrences alors que 100-X1 pourcents en ont trouvé plus », X1 étant un nombre réel compris entre 0 et 100, et N1 étant un nombre entier supérieur ou égal à 0 déterminé en fonction de l'examen. Dans un exemple d'application dans le cadre d'une analyse de lavage bronco alvéolaire (« LBA ») avec l'évaluation du profil cytologique qui nécessite une numération des différentes cellules du LBA dont celles des macrophages, le prérapport peut comprendre une phrase du type « l'analyse montre que le prélèvement comprend X2 pourcents de macrophages par rapport à la population cellulaire totale du prélèvement analysé ».

Pour la traçabilité de l'examen, le système peut permettre de savoir qui a trouvé le plus de ROI/OOI et qui en a trouvé moins.

Si les experts répondent différemment à une question, le prérapport peut comprendre, dans le chapitre dont dépend la question, un compte-rendu normé sous forme de phrases préétablies telles que, par exemple : « A la question de savoir si Y (prédéfinie par l'administrateur) était présent, n experts sur N2 ont répondu par l'affirmative alors de N2-n ont répondu par la négative », N2 étant le nombre total d'experts ayant participé au traitement de l'image, et n étant un entier compris entre 0 et N2.

C'est par exemple le cas dans le cadre d'un cancer du sein. L'analyse d'une biopsie pré-opératoire comprend, parmi la liste de questions qui vont orienter le traitement, la question « Nombre de mitoses dans une surface de **** (prédéfinie par l'administrateur) mm² », ce qui permettra d'établir l'index mitotique. Le prérapport peut alors comprendre, dans le chapitre dont dépend la question, une phrase du type : « Concernant le nombre de mitoses, le comptage de n experts sur N2 abouti à un indice mitotique de ... alors que le comptage de N2-n abouti à un indice mitotique de ... ».

Le système selon l'invention est donc capable de générer un document prérempli, reprenant le résultat des analyses statistiques des réponses et des annotations des experts, dans un ordre prédéterminé, sans risque d'oubli d'examen, ni de perte d'information. Cependant, le prérapport ne comprend aucun diagnostic, car il se contente d'exposer de manière normée et organisée les résultats statistiques de l'analyse automatique des annotations des différents experts.

Ce prérapport est ensuite complété par l'auteur en charge de la rédaction du rapport.

La normalisation du rapport ainsi que la traçabilité des données rendues possible par le système de traitement selon l'invention permettent de réaliser des examens à plusieurs pour en assurer la pertinence et l'efficacité, sans risque de perdre de l'information (risque inhérent à un examen à plusieurs) et avec une analyse statistique qui serait impossible sans le système selon l'invention.

En effet, sans le système selon l'invention, il faudrait analyser un nombre de données considérable, concernant des examens n'ayant pas eu lieu en même temps ni dans les mêmes conditions, à partir de comptes-rendus non homogènes. Outre la quantité de travail dissuasive que cela représente, le résultat d'une telle analyse serait fortement contestable puisque les données dépendent d'un trop grand nombre de paramètres.

Le système selon l'invention permet de réduire le nombre de ces paramètres de sorte que l'analyse des examens est rapide, facile et très significative.

Avantageusement, le pré rapport d'examen comprend un identifiant unique de l'image à traiter, l'image résultat, et le compte-rendu normé d'une analyse statistique de l'image résultat.

Selon d'autres modes de réalisation :
- Le système selon l'invention peut assurer l'anonymisation des données pour qu'aucun des experts ne connaisse les coordonnées du patient dont ils examinent un échantillon. Pour cela, le serveur distant comprend une première mémoire de stockage d'images originales associées chacune à des informations d'identification confidentielles des patients, une deuxième mémoire de stockage d'images à traiter correspondantes, dépourvues des informations d'identification confidentielles mais associées chacune à un identifiant unique, et un tableau de correspondance entre les images originales et les images à traiter. La suppression des informations confidentielles, la création d'un identifiant unique et la création de la table de données correspondante peuvent être faites automatiquement par le serveur distant programmé à cette fin.

L'unité centrale de la console est programmée pour ajouter à l'image résultat obtenue après annotation d'une image à traiter, l'identifiant unique de l'image à traiter et pour transmettre l'image résultat munie de l'identifiant unique au serveur distant.

Ce dernier pourra alors réattribuer à l'image résultat et au rapport d'examen les coordonnées du patient, de sorte que le médecin en charge du patient pourra rendre compte au patient des résultats de l'examen, sans risque de confusion de dossiers, et sans risque que les experts aient eu connaissance des coordonnées du patient.
- Le système selon l'invention peut également prévoir que la console d'un premier système selon l'invention soit en mesure de communiquer spécifiquement et de manière sécurisée avec une console d'un autre système selon l'invention installé à distance du premier et en communication réseau avec lui. Cet agencement permet des séances de travail entre deux ou plusieurs groupes d'utilisateurs (experts, élèves, etc.) distants.

Ainsi, le système selon l'invention comprend au moins deux consoles de distribution d'images à traiter et de collecte d'images annotées aptes à communiquer entre elles, les consoles étant programmées pour avoir un statut paramétrable permettant une organisation hiérarchique des consoles.

Dans ce cas, l'échange de données se fait entre au moins deux consoles, en respectant l'organisation hiérarchique des consoles. Ainsi, l'une des consoles présente un statut principal alors que les autres consoles présentent un statut secondaire, la préparation de la séance de travail étant faite sur la console principale et transmise aux consoles secondaires. Pendant l'examen, chaque console assure son rôle de centralisation des données du groupe dont elle dépend : elle gère l'affichage commandé au sein du groupe, génère une image résultat et une analyse statistique des données du groupe.

La console principale peut prendre la main sur les consoles secondaires, c'est-à-dire les commander, et permettre, par exemple, un affichage commandé par un membre d'un groupe pour tous les membres de tous les groupes.

Elle peut également soit générer une image résultat à partir des images annotées transmises directement par les consoles des autres groupes, soit générer une image résultat principale à partir des images résultats secondaires générées puis envoyées par chacune des consoles secondaires. Cette deuxième option présente l'avantage que la puissance de calcul est répartie entre toutes les consoles pour générer l'image résultat finale. Cependant, ce mode de réalisation dépend de la qualité de la communication entre les consoles des différents systèmes selon l'invention. Il reste très intéressant, car le volume de données échangé est restreint. Un réseau sans fil de type LoRa ou SigFox pourrait servir de réseau de communication.
- Les interfaces sont avantageusement identiques, ce qui permet d'assurer une l'homogénéité de visualisation sur les interfaces et réduit les erreurs de diagnostics imputables au matériel. Cela améliore donc la qualité de l'examen.
- Les interfaces utilisables dans le système selon l'invention peuvent être un terminal mobile, un ordinateur et son écran, une tablette, de préférence tactile, un dispositif de micro-affichage situé dans le plan focal de l'utilisateur, un casque de réalité virtuelle, etc.

Grâce à l'invention, les utilisateurs s'affranchissent des contraintes de performance de matériel et de débit de leur réseau de télécommunication, car la console n'a pas besoin d'une connexion réseau tierce pour fonctionner avec les interfaces en mode local.

En outre, le système prend en charge de multiples formats d'images. A contrario, les logiciels actuels fonctionnent avec un seul format d'images propriétaires (celui du fabricant de scanner, qui commercialise le logiciel).

En outre, le système selon l'invention est interopérable avec les systèmes de gestion de laboratoire déjà existant, tels que les PACS (pour « Picture Archiving and Communication System » en anglais ou Système d'Archivage et de Transmissions d'Images Médicales, utilisés dans les hôpitaux).

L'invention a été principalement décrite avec une mise en œuvre dans le domaine médical. Elle n'est absolument pas exclusive de ce domaine qui n'est déterminé que par le sujet des images.

L'invention peut parfaitement être utilisée dans le domaine industriel comme outil d'aide au diagnostic ou au contrôle de qualité.

L'invention peut également s'appliquer au domaine de la location de voiture, comme outil d'aide au diagnostic et de suivi de constat pour la location de voiture.

Grâce au système, le loueur et le locataire annotent chacun la même image du véhicule (photo ou plan) sur son interface (de préférence une tablette tactile) et la console compare les résultats avec un taux de similitude déterminé. Si les différences sont trop importantes, le constat n'est pas validé et les protagonistes sont invités à comparer leurs annotations et à les modifier pour que le taux de similitude soit suffisant. Dans ce cas, après avoir généré l'image résultat, la console renvoie une alerte ainsi qu'une image (leur image traitée, de préférence) comprenant les points de différence sur chaque interface. Avantageusement, la console affiche également de manière dynamique sur chaque interface une valeur de similitude avec un code couleur : valeur du taux et format en rouge si le taux de similitude est insuffisant. Les protagonistes s'entendent alors et modifient leur image traitée. Tout au long des modifications d'annotation, la console calcule de manière interactive et dynamique le taux de similitude des deux images à nouveau traitées et l'affiche sur chaque console jusqu'à ce que le taux de similitude soit suffisant. La console affiche alors un signal (par exemple un taux de similitude en vert) et le constat est validé.

Il est ensuite stocké et peut être comparé au constat précédent en cas de litige. Grâce à l'utilisation d'un guide d'annotation séquentiel accompagné d'un référentiel terminologique et de marques d'annotation pré-réglées, la réalisation du constat est rapide et sûre, et permet un historique fiable permettant d'identifier rapidement les responsabilités de chacun.

## Revendications

1. Système (100) de traitement d'image collaboratif et interactif pour un domaine nécessitant un partage d'avis et d'analyse graphique d'images pour limiter les risques d'erreur d'appréciation individuels, **caractérisé en ce qu'**il comprend :
• une console de distribution (110) d'images à traiter (200) et de collecte d'images annotées (201a, 201b, 201c, 201d, 201e) apte à communiquer localement avec au moins deux interfaces de traitement (120) aptes à afficher une image à traiter,
• **en ce que** la console et les interfaces (120) sont programmées :
o pour transmettre une copie de l'image à traiter sur chaque interface de traitement (120) depuis la console de distribution (110), l'image à traiter étant associée à un type d'examen, le type d'examen étant associé à une liste des objets d'intérêt et/ou régions d'intérêt, et chaque objet d'intérêt et/ou région d'intérêt étant associé à une marque d'annotation,
o pour fournir des outils (B3, B3a, B3b) d'annotation graphique de la copie de l'image sur chaque interface pour obtenir une image annotée avec des modifications graphique, les outils d'annotation graphique étant prédéfinis pour l'image et permettent de délimiter graphiquement les régions d'intérêt et/ou ajouter une marque graphique sur les objets d'intérêt prédéfinis,
o pour transmettre chaque image annotée sur la console de distribution (110) depuis chaque interface de traitement (120),
o et pour générer une image résultat à partir des images annotées à l'aide d'un algorithme combinatoire analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes,
• et **en ce que** la console (110) et les interfaces (120) sont programmées
o pour permettre un affichage selon un mode individuel dans lequel l'affichage de chaque interface est indépendant des autres interfaces,
o et pour permettre un affichage selon un mode commandé dans lequel l'affichage d'une première interface commande l'affichage sur les autres interfaces, le passage entre le mode individuel et le mode commandé étant réversible..

2. Système de traitement d'image selon la revendication 1, dans lequel la console (110) et les interfaces (120) sont programmées pour permettre de marquer sur la copie de l'image à traiter au moins une région d'intérêt (300, 301, 302, 303) définie par un identifiant unique, une forme représentative de la région d'intérêt et des coordonnées dans la copie de l'image à traiter.

3. Système de traitement d'image collaboratif et interactif selon l'une quelconque des revendications 1 ou 2, comprenant :
• Une console (110) de distribution et de collecte de données comprenant :
- un émetteur/récepteur sans fil (111) d'un signal sans-fil local ;
- une mémoire de stockage pour au moins une image à traiter et pour au moins une image résultat ;
- une unité centrale programmée ;
• au moins deux interfaces de traitement (120) comprenant :
- un émetteur/récepteur sans fil d'un signal sans-fil local compatible avec l'émetteur/récepteur de la console pour permettre un échange local de données via le signal sans fil ;
- une mémoire de stockage pour au moins une image à traiter et pour au moins une image annotée ;
- une unité centrale programmée.

4. Système de traitement d'image collaboratif et interactif selon la revendication 3, dans lequel l'unité centrale de la console (110) et l'unité centrale de chaque interface (120) sont programmées pour :
- transmettre une copie d'au moins une image à traiter à toutes les interfaces ;
- afficher une image à traiter entière sur chaque interface,
- agrandir et/ou rétrécir sur commande l'image affichée et mémoriser une valeur de zoom représentative de l'agrandissement de l'image affichée par rapport à l'image entière ainsi qu'une position de l'image affichée par rapport à l'image entière ;
- exécuter, à partir d'une première interface, un affichage selon un mode commandé réversible de l'affichage des autres interfaces, en commandant sur toutes les autres interfaces un affichage identique à l'affichage de la première interface ;
- apposer au moins une marque graphique d'annotation sur l'image à traiter pour générer une image annotée ;
- enregistrer et transmettre de chaque image annotée sur la console avec un identifiant représentatif de l'interface ayant transmis l'image annotée.

5. Système de traitement d'image selon la revendication 4, dans lequel si l'affichage en mode commandé est activé par un utilisateur, l'unité centrale de la console et l'unité centrale de chaque interface sont programmées pour mémoriser préalablement un affichage initial de chaque interface, pour commander sur toutes les autres interfaces un affichage identique à l'affichage de la première interface, puis pour réafficher l'affichage initial de chaque interface lorsque le mode commandé est désactivé.

6. Système de traitement d'image selon l'une quelconque des revendications 3 à 5, dans lequel l'unité centrale de la console est programmée pour combiner les images annotées envoyées par chaque interface et générer une image résultat avec un algorithme combinatoire analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes.

7. Système de traitement d'image selon la revendication 6, dans lequel l'unité centrale de la console est programmée pour générer une image résultat (400) dans laquelle des marques d'annotations graphiques (301-302) des images annotées présentant un taux de similitude supérieur à un seuil de similitude déterminé sont affichées selon un premier format graphique, et dans laquelle des marques d'annotations graphiques (303, M1, M2, M3, M4) des images annotées présentant un taux de similitude inférieur au seuil de similitude déterminé sont affichées selon au moins un deuxième format graphique.

8. Système de traitement d'image selon la revendication 7 dans lequel l'unité centrale de la console est programmée pour exécuter une analyse statistique des marques d'annotations graphiques.

9. Système de traitement d'image selon la revendication 8, dans lequel l'analyse statistique comprend un calcul de pourcentage de marques d'annotations graphiques présentant un taux de similitude supérieur à un seuil de similitude déterminé et un calcul de pourcentage de marques d'annotations graphiques présentant un taux de similitude inférieur au seuil de similitude déterminé.

10. Système de traitement d'image selon l'une quelconque des revendications 7 à 9, dans lequel le seuil de similitude est paramétrable.

11. Système de traitement d'image selon l'une quelconque des revendications 7 à 10, dans lequel l'unité centrale de la console et/ou de chaque interface est également programmée pour générer une table de correspondance entre chaque marque d'annotation graphique, définie au moins par des coordonnées de position dans l'image annotée, et son auteur.

12. Système de traitement d'image selon la revendication 11, dans lequel l'unité centrale de la console est également programmée afficher un identifiant d'auteur de chaque marque d'annotation graphique sur l'image résultat.

13. Système de traitement d'image selon l'une quelconque des revendications 3 à 12, dans lequel l'unité centrale de chaque interface est programmée pour être activable avec un code administrateur ou avec un code utilisateur, l'unité centrale de la console étant programmée pour afficher sur une interface activée avec le code administrateur, des outils d'administration comprenant : une liste d'images à traiter présentes dans la mémoire de l'unité centrale, une liste d'identifiants des participants, une liste pour des descripteurs d'objets ou de région d'intérêt sélectionnables par les utilisateurs, chaque descripteur étant associés à une marque d'annotation graphique prédéfinie différente.

14. Système de traitement d'image selon la revendication 13, dans lequel la console comprend une prise de connexion (112) à une mémoire amovible reliée à l'unité centrale de la console.

15. Système de traitement d'image selon l'une quelconque des revendications 13 ou 14, dans lequel la console comprend une prise de connexion réseau reliée à l'unité centrale de la console (110), cette dernière étant programmée pour communiquer avec un serveur distant (140) via un réseau de communication pour télécharger au moins une image à traiter et à la stocker en mémoire.

16. Système de traitement d'image selon la revendication 15, dans lequel le serveur distant (140) comprend une première mémoire de stockage d'images originales associées chacune à des informations d'identification confidentielles, une deuxième mémoire de stockage d'images à traiter correspondantes, dépourvues des informations d'identification confidentielles mais associées chacune à un identifiant unique, et un tableau de correspondance entre les images originales et les images à traiter.

17. Système de traitement d'image selon la revendication 16, dans lequel l'unité centrale de la console est programmée pour ajouter à l'image résultat obtenue après annotation d'une image à traiter l'identifiant unique de l'image à traiter et pour transmettre l'image résultat munie de l'identifiant unique au serveur distant.

18. Système de traitement d'image selon l'une quelconque des revendications 3 à 17, dans lequel l'unité centrale de la console et l'unité centrale des interfaces sont programmées pour afficher un guide d'annotation séquentiel accompagné d'un référentiel terminologique, pour enregistrer séquentiellement les réponses de chaque expert, et pour générer un pré rapport d'examen comprenant un plan d'examen de l'image à traiter dans lequel les réponses de chaque expert sont reportées et combinées séquentiellement.

19. Système de traitement d'image selon la revendication 18, dans lequel le pré rapport d'examen comprend un identifiant unique de l'image à traiter, l'image résultat, et un compte-rendu normé d'une analyse statistique de l'image résultat.

20. Système de traitement d'image selon l'une quelconque des revendications 1 à 19, comprenant au moins deux consoles de distribution (110) d'images à traiter (200) et de collecte d'images annotées (201a, 201b, 201c, 201d, 201e) aptes à communiquer entre elles, les consoles étant programmées pour avoir un statut paramétrable permettant une organisation hiérarchique des consoles.

21. Système de traitement d'image selon la revendication 20, dans lequel l'une des consoles est programmée pour présenter un statut principal alors que les autres consoles présentent un statut secondaire, la console présentant un statut principal étant apte à commander les consoles présentant un statut secondaire.

22. Procédé de traitement d'image collaboratif et participatif pour un domaine nécessitant un partage d'avis et d'analyse graphique d'images pour limiter les risques d'erreur d'appréciation individuels, **caractérisé en ce qu'**il comprend les étapes suivantes :
- placer une image à traiter (200) dans une mémoire d'une console (110) apte à communiquer localement avec au moins deux interfaces de traitement (120) ;
- transmettre une copie de l'image à traiter à chaque interface de traitement depuis la console de distribution, l'image à traiter étant associée à un type d'examen, le type d'examen étant associé à une liste des objets d'intérêt et/ou régions d'intérêt, et chaque objet d'intérêt et/ou région d'intérêt étant associé à une marque d'annotation ;
- sur chaque interface de traitement, afficher des outils d'annotation graphique (B3, B3a, B3b) de la copie de l'image à traiter pour permettre à un utilisateur de modifier graphiquement la copie de l'image à traiter et ainsi générer une image annotée (201a, 201b, 201c, 201d, 201e), les outils d'annotation graphique étant prédéfinis pour l'image et permettent de délimiter graphiquement les régions d'intérêt et/ou ajouter une marque graphique sur les objets d'intérêt prédéfinis ;
- permettre un affichage selon un mode individuel dans lequel l'affichage de chaque interface est indépendant des autres interfaces, et
- permettre un affichage selon un mode commandé dans lequel l'affichage d'une première interface commande l'affichage sur les autres interfaces, le passage entre le mode individuel et le mode commandé étant réversible;
- transmettre chaque image annotée sur la console de distribution depuis chaque interface de traitement, et
- générer avec la console de distribution une image résultat à partir des images annotées à l'aide d'un algorithme combinatoire déterminé analysant les modifications graphiques de chaque image annotée pour déterminer les modifications graphiques similaires et les modifications graphiques différentes entre les images annotées, et synthétisant cette analyse en générant une image résultat constituée de pixels de formats graphiques différents entre les modifications graphiques similaires et les modifications graphiques différentes.

23. Procédé de traitement d'image selon la revendication 22, dans lequel les annotations graphiques des images annotées présentant un taux de similitude supérieur à un seuil de similitude déterminé sont marquées selon un premier format graphique dans l'image résultat, et dans lequel les annotations graphiques de différentes images présentant un taux de similitude inférieur au seuil de similitude déterminé sont marquées selon au moins un deuxième format graphique dans l'image résultat.

## Patentansprüche

1. Kollaboratives und interaktives Bildverarbeitungssystem (100) für einen Bereich, der eine Teilung von Nachrichten und von grafischer Bilderanalyse erfordert, um die individuellen Risiken eines Beurteilungsfehlers zu begrenzen,
• eine Konsole (110) für das Verteilen von zu verarbeitenden Bildern (200) und für das Sammeln von annotierten Bildern (201a, 201b, 201c, 201d, 201e), die geeignet ist, lokal mit mindestens zwei Verarbeitungs-schnittstellen (120) zu kommunizieren, die geeignet sind, ein zu verarbeitendes Bild anzuzeigen,
• dass die Konsole und die Schnittstellen (120) programmiert sind, um:
∘ eine Kopie des zu verarbeitenden Bildes von der Verteilungskonsole (110) auf jede Verarbeitungsschnittstelle (120) zu übertragen, wobei das zu verarbeitende Bild einem Prüfungstyp zugeordnet ist, wobei der Prüfungstyp einer Liste der interessierenden Objekte und/oder interessierenden Gebiete zugeordnet ist und wobei das jeweilige interessierende Objekt und/oder interessierende Gebiet einer Annotationsmarke zugeordnet ist,
∘ Werkzeuge (B3, B3a, B3b) für die grafische Annotierung der Kopie des Bildes auf jeder Schnittstelle bereitzustellen, um ein annotiertes Bild mit grafischen Änderungen zu erzielen, wobei die Werkzeuge für die grafische Annotierung für das Bild vorbestimmt sind und es ermöglichen, die interessierenden Gebiete grafisch abzugrenzen und/oder eine grafische Marke auf den vorbestimmten interessierenden Objekten hinzuzufügen,
o das jeweilige annotierte Bild von der jeweiligen Verarbeitungsschnittstelle (120) auf die Verteilungskonsole (110) zu übertragen, und
o ein Ergebnisbild zu erzeugen ausgehend von den annotierten Bildern mittels eines kombinatorischen Algorithmus, der die grafischen Änderungen des jeweiligen annotierten Bildes analysiert, um die ähnlichen grafischen Änderungen und die unterschiedlichen grafischen Änderungen zwischen den annotierten Bildern zu bestimmen, und der diese Analyse synthetisiert, indem ein Ergebnisbild bestehend aus Pixeln mit unterschiedlichen grafischen Formaten zwischen den ähnlichen grafischen Änderungen und den unterschiedlichen grafischen Änderungen erzeugt wird, und
• dass die Konsole (110) und die Schnittstellen (120) programmiert sind, um:
o eine Anzeige gemäß einem individuellen Modus zu ermöglichen, in dem die Anzeige der jeweiligen Schnittstelle von den anderen Schnittstellen unabhängig ist, und
o eine Anzeige gemäß einem gesteuerten Modus zu ermöglichen, in dem die Anzeige einer ersten Schnittstelle die Anzeige auf den anderen Schnittstellen steuert, wobei der Wechsel zwischen individuellem Modus und gesteuertem Modus reversibel ist.

2. Bildverarbeitungssystem nach Anspruch 1, bei dem die Konsole (110) und die Schnittstellen (120) programmiert sind, um es zu ermöglichen, auf der Kopie des zu verarbeitenden Bildes mindestens ein durch eine eindeutige Kennung definiertes interessierendes Gebiet (300, 301, 302, 303), eine für das interessierende Gebiet repräsentative Form und Koordinaten in der Kopie des zu verarbeitenden Bildes zu markieren.

3. Kollaboratives und interaktives Bildverarbeitungssystem nach einem der Ansprüche 1 oder 2, umfassend:
• eine Datenverteilungs- und -sammlungskonsole (110) mit:
- einem drahtlosen Sender-Empfänger (111) für ein drahtloses lokales Signal,
- - einem Speicher für die Speicherung mindestens eines zu verarbeitenden Bildes und mindestens eines Ergebnisbildes,
- einer CPU,
• mindestens zwei Verarbeitungsschnittstellen (120) mit:
- einem drahtlosen Sender-Empfänger für ein drahtloses lokales Signal, der mit dem Sender-Empfänger der Konsole kompatibel ist, um einen lokalen Datenaustausch über das drahtlose Signal zu ermöglichen,
- einem Speicher für die Speicherung mindestens eines zu verarbeitenden Bildes und mindestens eines annotierten Bildes,
- einer CPU.

4. Kollaboratives und interaktives Bildverarbeitungssystem nach Anspruch 3,
bei dem die zentrale Einheit der Konsole (110) und die zentrale Einheit der jeweiligen Schnittstelle (120) programmiert sind, um:
- eine Kopie mindestens eines zu verarbeitenden Bildes an alle Schnittstellen zu übertragen,
- ein ganzes zu verarbeitendes Bild auf der jeweiligen Schnittstelle anzuzeigen,
- das angezeigte Bild auf Befehl zu vergrößern und/oder zu verengen und einen für die Vergrößerung des angezeigten Bildes im Verhältnis zum ganzen Bild repräsentativen Zoom-Wert sowie eine Position des angezeigten Bildes in Bezug auf das ganze Bild zu speichern,
- von einer ersten Schnittstelle aus eine Anzeige gemäß einem reversiblen gesteuerten Modus der Anzeige der anderen Schnittstellen durchzuführen, indem auf allen anderen Schnittstellen eine Anzeige gesteuert wird, die identisch ist mit der Anzeige auf der ersten Schnittstelle,
- mindestens eine grafische Annotationsmarke auf das zu verarbeitende Bild anzubringen, um ein annotiertes Bild zu erzeugen,
- das jeweilige annotierte Bild aufzuzeichnen und auf die Konsole mit einer Kennung zu übertragen, die für die Schnittstelle repräsentativ ist, die das annotierte Bild übertragen hat.

5. Bildverarbeitungssystem nach Anspruch 4, bei dem, wenn die Anzeige im gesteuerten Modus durch einen Benutzer aktiviert wird, die zentrale Einheit der Konsole und die zentrale Einheit der jeweiligen Schnittstelle programmiert sind, um eine ursprüngliche Anzeige der jeweiligen Schnittstelle vorabzuspeichern, um auf allen anderen Schnittstellen eine Anzeige zu steuern, die identisch ist mit der Anzeige der ersten Schnittstellen, und um anschließend die ursprüngliche Anzeige der jeweiligen Schnittstelle erneut anzuzeigen, wenn der gesteuerte Modus deaktiviert ist.

6. Bildverarbeitungssystem nach einem der Ansprüche 3 bis 5, bei dem die zentrale Einheit der Konsole programmiert ist, um die von der jeweiligen Schnittstelle gesendeten annotierten Bilder zu kombinieren und um ein Ergebnisbild mit einem kombinatorischen Algorithmus zu erzeugen, der die grafischen Änderungen des jeweiligen annotierten Bildes analysiert, um die ähnlichen grafischen Änderungen und die unterschiedlichen grafischen Änderungen zwischen den annotierten Bildern zu bestimmen, und der diese Analyse synthetisiert, indem ein Ergebnisbild bestehend aus Pixeln mit unterschiedlichen grafischen Formaten zwischen den ähnlichen grafischen Änderungen und den unterschiedlichen grafischen Änderungen erzeugt wird.

7. Bildverarbeitungssystem nach Anspruch 6, bei dem die zentrale Einheit der Konsole programmiert ist, um ein Ergebnisbild (400) zu erzeugen, in dem grafische Annotationsmarken (301-302) der annotierten Bilder mit einer eine bestimmte Ähnlichkeitsschwelle überschreitenden Ähnlichkeitsrate gemäß einem ersten grafischen Format angezeigt werden und in dem grafische Annotationsmarken (303, M1, M2, M3, M4) der annotierten Bilder mit einer die bestimmte Ähnlichkeitsschwelle unterschreitenden Ähnlichkeitsrate gemäß mindestens einem zweiten grafischen Format angezeigt werden.

8. Bildverarbeitungssystem nach Anspruch 7, bei dem die zentrale Einheit der Konsole programmiert ist, um eine statistische Analyse der grafischen Annotationsmarken durchzuführen.

9. Bildverarbeitungssystem nach Anspruch 8, bei dem die statistische Analyse eine Prozentrechnung von grafischen Annotationsmarken mit einer eine bestimmte Ähnlichkeitsschwelle überschreitenden Ähnlichkeitsrate und eine Prozentrechnung von grafischen Annotationsmarken mit einer die bestimmte Ähnlichkeitsschwelle unterschreitenden Ähnlichkeitsrate umfasst.

10. Bildverarbeitungssystem nach einem der Ansprüche 7 bis 9, bei dem die Ähnlichkeitsschwelle einstellbar ist.

11. Bildverarbeitungssystem nach einem der Ansprüche 7 bis 10, bei dem die zentrale Einheit der Konsole und/oder der jeweiligen Schnittstelle ebenfalls programmiert ist, um eine Entsprechungstabelle zwischen der jeweiligen grafischen Annotationsmarke, welche mindestens durch Positionskoordinaten in dem annotierten Bild definiert ist, und ihrem Urheber zu erzeugen.

12. Bildverarbeitungssystem nach Anspruch 11, bei dem die zentrale Einheit der Konsole ebenfalls programmiert ist, um eine Urheberkennung der jeweiligen grafischen Annotationsmarke auf dem Ergebnisbild anzuzeigen.

13. Bildverarbeitungssystem nach einem der Ansprüche 3 bis 12, bei dem die zentrale Einheit der jeweiligen Schnittstelle programmiert ist, um mit einem Administratorcode oder einem Benutzercode aktivierbar zu sein, wobei die zentrale Einheit der Konsole programmiert ist, um auf einer mit dem Administratorcode aktivierten Schnittstelle Administrationswerkzeuge anzuzeigen, welche umfassen:
eine Liste zu verarbeitender Bilder, die in dem Speicher der zentralen Einheit vorhanden sind, eine Liste von Kennungen der Teilnehmer, eine Liste für Deskriptoren von interessierenden Objekten oder Gebieten, die von den Benutzern ausgewählt werden können, wobei jeder Deskriptor einer unterschiedlichen vorbestimmten grafischen Annotations-marke zugeordnet ist.

14. Bildverarbeitungssystem nach Anspruch 13, bei dem die Konsole eine Buchse (112) für den Anschluss an einen abnehmbaren, mit der zentralen Einheit der Konsole verbundenen Speicher umfasst.

15. Bildverarbeitungssystem nach einem der Ansprüche 13 oder 14, bei dem die Konsole eine Netzwerkverbindungsbuchse umfasst, die mit der zentralen Einheit der Konsole (110) verbunden ist, wobei Letztere programmiert ist, um mit einem entfernten Server (140) über ein Kommunikationsnetzwerk zu kommunizieren, um mindestens ein zu verarbeitendes Bild herunterzuladen und es zu speichern.

16. Bildverarbeitungssystem nach Anspruch 15, bei dem der entfernte Server (140) einen ersten Speicher für Originalbilder, die jeweils vertraulichen Identifizierungsinformationen zugeordnet sind, einen zweiten Speicher für entsprechende zu verarbeitende Bilder ohne die vertraulichen Identifizierungsinformationen, die aber jeweils einer eindeutigen Kennung zugeordnet sind, und eine Entsprechungstabelle zwischen den Originalbildern und den zu verarbeitenden Bildern, umfasst.

17. Bildverarbeitungssystem nach Anspruch 16, bei dem die zentrale Einheit der Konsole programmiert ist, um dem nach Annotierung eines zu verarbeitenden Bildes erhaltenen Ergebnisbild die eindeutige Kennung des zu verarbeitenden Bildes hinzuzufügen und um das mit der eindeutigen Kennung versehene Ergebnisbild an den entfernten Server zu übertragen.

18. Bildverarbeitungssystem nach einem der Ansprüche 3 bis 17, bei dem die zentrale Einheit der Konsole und die zentrale Einheit der Schnittstellen programmiert sind, um einen sequentiellen Annotationsleiter mit einer Terminologiedatenbank anzuzeigen, um die Antworten des jeweiligen Experten sequentiell aufzuzeichnen und um einen Vorab-Prüfbericht mit einem Prüfungsplan des zu verarbeitenden Bildes zu erzeugen, in dem die Antworten des jeweiligen Experten sequentiell übertragen und kombiniert werden.

19. Bildverarbeitungssystem nach Anspruch 18, bei dem der Vorab-Prüfbericht eine eindeutige Kennung des zu verarbeitenden Bildes, das Ergebnisbild und einen normierten Bericht einer statistischen Analyse des Ergebnisbildes enthält.

20. Bildverarbeitungssystem nach einem der Ansprüche 1 bis 19, umfassend mindestens zwei Konsolen (110) für das Verteilen von zu verarbeitenden Bildern (200) und das Sammeln annotierter Bilder (201a, 201b, 201c, 201d, 201e), welche geeignet sind, miteinander zu kommunizieren, wobei die Konsolen programmiert sind, um einen einstellbaren Status aufzuweisen, der eine hierarchische Organisation der Konsolen ermöglicht.

21. Bildverarbeitungssystem nach Anspruch 20, bei dem eine der Konsolen programmiert ist, um einen Hauptstatus aufzuweisen, während die anderen Konsolen einen Nebenstatus aufweisen, wobei die Konsole mit einem Hauptstatus geeignet ist, die Konsolen mit einem Nebenstatus zu steuern.

22. Kollaboratives und partizipatives Bildverarbeitungsverfahren für einen Bereich, der eine Teilung von Nachrichten und von grafischer Analyse von Bildern erfordert, um die individuellen Risiken eines Beurteilungsfehlers zu begrenzen, **dadurch gekennzeichnet, dass** es die folgenden Schritte umfasst:
- ein zu verarbeitendes Bild (200) in einen Speicher einer Konsole (110) setzen, die geeignet ist, lokal mit mindestens zwei Verarbeitungsschnittstellen (120) zu kommunizieren,
- eine Kopie des zu verarbeitenden Bildes von der Verteilungskonsole an jede Verarbeitungsschnittstelle übertragen, **wobei** das zu verarbeitende Bild einem Prüfungstyp zugeordnet ist, **wobei** der Prüfungstyp einer Liste der interessierenden Objekte und/oder interessierenden Gebiete zugeordnet ist und **wobei** das jeweilige interessierende Objekt und/oder interessierende Gebiet einer Annotationsmarke zugeordnet ist,
- auf jeder Verarbeitungsschnittstelle Werkzeuge (B3, B3a, B3b) für die grafische Annotierung der Kopie des zu verarbeitenden Bildes anzeigen, um es einem Benutzer zu ermöglichen, die Kopie des zu verarbeitenden Bildes grafisch zu ändern und somit ein annotiertes Bild (201a, 201b, 201c, 201d, 201e) zu erzeugen, **wobei** die Werkzeuge für grafische Annotierung für das Bild vorbestimmt sind und es ermöglichen, die interessierenden Gebiete grafisch abzugrenzen und/oder eine grafische Marke auf den vorbestimmten interessierenden Objekten hinzuzufügen,
- eine Anzeige gemäß einem individuellen Modus ermöglichen, in dem die Anzeige der jeweiligen Schnittstelle unabhängig von den anderen Schnittstellen ist, und
- eine Anzeige gemäß einem gesteuerten Modus ermöglichen, in dem die Anzeige einer ersten Schnittstelle die Anzeige auf den anderen Schnittstellen steuert, wobei der Wechsel zwischen individuellem Modus und gesteuertem Modus reversibel ist,
- das jeweilige annotierte Bild von der jeweiligen Verarbeitungsschnittstelle auf die Verteilungskonsole übertragen und
- mit der Verteilungskonsole ein Ergebnisbild erzeugen ausgehend von den annotierten Bildern mittels eines bestimmten kombinatorischen Algorithmus, der die grafischen Änderungen des jeweiligen annotierten Bildes analysiert, um die ähnlichen grafischen Änderungen und die unterschiedlichen grafischen Änderungen zwischen den annotierten Bildern zu bestimmen, und der diese Analyse synthetisiert, indem ein Ergebnisbild bestehend aus Pixeln mit unterschiedlichen grafischen Formaten zwischen den ähnlichen grafischen Änderungen und den unterschiedlichen grafischen Änderungen erzeugt wird.

23. Bildverarbeitungsverfahren nach Anspruch 22, bei dem die grafischen Annotationen der annotierten Bilder mit einer eine bestimmte Ähnlichkeitsschwelle überschreitenden Ähnlichkeitsrate gemäß einem ersten grafischen Format in dem Ergebnisbild markiert werden und bei dem die grafischen Annotationen unterschiedlicher Bilder mit einer die bestimmte Ähnlichkeitsschwelle unterschreitenden Ähnlichkeitsrate gemäß mindestens einem zweiten grafischen Format in dem Ergebnisbild markiert werden.

## Claims

1. Collaborative and interactive image processing system (100) for a field requiring the sharing of views and of graphical analysis of images in order to limit individual risks of error of judgement, **characterized in that** it comprises:
• a console (110) for distributing images (200) to be processed and for collecting annotated images (201a, 201b, 201c, 201d, 201e), able to communicate locally with at least two processing interfaces (120) that are able to display an image to be processed,
• **in that** the console and the interfaces (120) are programmed:
∘ to transmit a copy of the image to be processed to each processing interface (120) from the distribution console (110), the image to be processed being associated with a type of examination, the type of examination being associated with a list of objects of interest and/or regions of interest, and each object of interest and/or region of interest being associated with an annotation mark,
∘ to provide tools (B3, B3a, B3b) for graphically annotating the copy of the image on each interface in order to obtain an image annotated with graphical modifications, the graphical annotation tools being predefined for the image and allowing the regions of interest to be graphically delimited and/or a graphical mark to be added to the predefined objects of interest,
∘ to transmit each annotated image to the distribution console (110) from each processing interface (120),
∘ and to generate a result image from the annotated images using a combinatorial algorithm analysing the graphical modifications of each annotated image in order to determine similar graphical modifications and different graphical modifications between the annotated images, and synthesizing this analysis by generating a result image consisting of pixels of graphical formats that differ between the similar graphical modifications and the different graphical modifications,
• and **in that** the console (110) and the interfaces (120) are programmed
∘ to allow a display in an individual mode in which the display of each interface is independent of the other interfaces,
∘ and to allow a display in a commanded mode in which the display of a first interface commands the display on the other interfaces, the change between the individual mode and the commanded mode being reversible.

2. Image processing system according to Claim 1, wherein the console (110) and the interfaces (120) are programmed to make it possible to mark, on the copy of the image to be processed, at least one region of interest (300, 301, 302, 303) defined by a unique identifier, a shape representative of the region of interest and coordinates in the copy of the image to be processed.

3. Collaborative and interactive image processing system according to either one of Claims 1 and 2, comprising:
• a data distribution and collection console (110) comprising:
- a wireless transceiver (111) for transmitting/receiving a local wireless signal;
- a memory for storing at least one image to be processed and at least one result image;
- a programmed central processing unit;
• at least two processing interfaces (120) comprising:
- a wireless transceiver for transmitting/receiving a local wireless signal compatible with the transceiver of the console for allowing a local exchange of data via the wireless signal;
- a memory for storing at least one image to be processed and at least one annotated image;
- a programmed central processing unit.

4. Collaborative and interactive image processing system according to Claim 3, wherein the central processing unit of the console (110) and the central processing unit of each interface (120) are programmed to:
- transmit a copy of at least one image to be processed to all of the interfaces;
- display a whole image to be processed on each interface,
- zoom in and/or zoom out on the displayed image on command and store a zoom value representative of the zoom level of the displayed image with respect to the whole image and a position of the displayed image with respect to the whole image;
- execute, from a first interface, a display in a reversible commanded mode of displaying the other interfaces, by commanding a display identical to the display of the first interface on all of the other interfaces;
- affix at least one graphical annotation mark on the image to be processed in order to generate an annotated image;
- record and transmit each annotated image to the console with an identifier representative of the interface that transmitted the annotated image.

5. Image processing system according to Claim 4, wherein, if the display in commanded mode is activated by a user, the central processing unit of the console and the central processing unit of each interface are programmed to store an initial display of each interface beforehand, to command a display identical to the display of the first interface on all of the other interfaces, and then to redisplay the initial display of each interface when the commanded mode is deactivated.

6. Image processing system according to any one of Claims 3 to 5, wherein the central processing unit of the console is programmed to combine the annotated images sent by each interface and generate a result image with a combinatorial algorithm analysing the graphical modifications of each annotated image in order to determine similar graphical modifications and different graphical modifications between the annotated images, and synthesizing this analysis by generating a result image consisting of pixels of graphical formats that differ between the similar graphical modifications and the different graphical modifications.

7. Image processing system according to Claim 6, wherein the central processing unit of the console is programmed to generate a result image (400) in which graphical annotation marks (301-302) on the annotated images having a similarity level above a determined similarity threshold are displayed in a first graphical format, and in which graphical annotation marks (303, M1, M2, M3, M4) on the annotated images having a similarity level below the determined similarity threshold are displayed in at least one second graphical format.

8. Image processing system according to Claim 7, wherein the central processing unit of the console is programmed to execute statistical analysis of the graphical annotation marks.

9. Image processing system according to Claim 8, wherein the statistical analysis comprises calculating a percentage of graphical annotation marks having a similarity level above a determined similarity threshold and calculating a percentage of graphical annotation marks having a similarity level below the determined similarity threshold.

10. Image processing system according to any one of Claims 7 to 9, wherein the similarity threshold is settable.

11. Image processing system according to any one of Claims 7 to 10, wherein the central processing unit of the console and/or of each interface is also programmed to generate a table of correspondence between each graphical annotation mark, defined at least by position coordinates in the annotated image, and its author.

12. Image processing system according to Claim 11, wherein the central processing unit of the console is also programmed to display an author identifier for each graphical annotation mark on the result image.

13. Image processing system according to any one of Claims 3 to 12, wherein the central processing unit of each interface is programmed to be able to be activated with an administrator code or with a user code, the central processing unit of the console being programmed to display, on an interface activated with the administrator code, administration tools comprising: a list of images to be processed that are present in the memory of the central processing unit, a list of identifiers of the participants, a list for descriptors of objects or of regions of interest which can be selected by users, each descriptor being associated with a different predefined graphical annotation mark.

14. Image processing system according to Claim 13, wherein the console comprises a connection port (112) for connecting to a removable memory connected to the central processing unit of the console.

15. Image processing system according to either one of Claims 13 and 14, wherein the console comprises a network connection port connected to the central processing unit of the console (110), said console being programmed to communicate with a remote server (140) via a communication network in order to download at least one image to be processed and to store it in memory.

16. Image processing system according to Claim 15, wherein the remote server (140) comprises a first memory for storing original images each associated with confidential identification information, a second memory for storing corresponding images to be processed, without confidential identification information but each associated with a unique identifier, and a table of correspondence between the original images and the images to be processed.

17. Image processing system according to Claim 16, wherein the central processing unit of the console is programmed to add, to the result image obtained after annotating an image to be processed, the unique identifier of the image to be processed, and to transmit the result image provided with the unique identifier to the remote server.

18. Image processing system according to any one of Claims 3 to 17, wherein the central processing unit of the console and the central processing unit of the interfaces are programmed to display a sequential annotation guide accompanied by a terminology database, to sequentially record the responses of each expert, and to generate a preliminary examination report comprising an examination layout for the image to be processed in which the responses of each expert are reported and combined sequentially.

19. Image processing system according to Claim 18, wherein the preliminary examination report comprises a unique identifier of the image to be processed, the result image, and a standardized record of statistical analysis of the result image.

20. Image processing system according to any one of Claims 1 to 19, comprising at least two consoles (110) for distributing images (200) to be processed and for collecting annotated images (201a, 201b, 201c, 201d, 201e) that are able to communicate with one another, the consoles being programmed to have a settable status allowing the consoles to be organized hierarchically.

21. Image processing system according to Claim 20, wherein one of the consoles is programmed to have a primary status, whereas the other consoles have a secondary status, the console having a primary status being able to command the consoles having a secondary status.

22. Collaborative and participative image processing method for a field requiring the sharing of views and of graphical analysis of images in order to limit individual risks of error of judgement, **characterized in that** it comprises the following steps:
- placing an image (200) to be processed in a memory of a console (110) able to communicate locally with at least two processing interfaces (120);
- transmitting a copy of the image to be processed to each processing interface from the distribution console, the image to be processed being associated with a type of examination, the type of examination being associated with a list of objects of interest and/or regions of interest, and each object of interest and/or region of interest being associated with an annotation mark;
- on each processing interface, displaying tools (B3, B3a, B3b) for graphically annotating the copy of the image to be processed in order to allow a user to graphically modify the copy of the image to be processed and thus generate an annotated image (201a, 201b, 201c, 201d, 201e), the graphical annotation tools being predefined for the image and allowing the regions of interest to be graphically delimited and/or a graphical mark to be added to the predefined objects of interest;
- allowing a display in an individual mode in which the display of each interface is independent of the other interfaces, and
- allowing a display in a commanded mode in which the display of a first interface commands the display on the other interfaces, the change between the individual mode and the commanded mode being reversible;
- transmitting each annotated image to the distribution console from each processing interface, and
- using the distribution console to generate a result image from the annotated images using a determined combinatorial algorithm analysing the graphical modifications of each annotated image in order to determine similar graphical modifications and different graphical modifications between the annotated images, and synthesizing this analysis by generating a result image consisting of pixels of graphical formats that differ between the similar graphical modifications and the different graphical modifications.

23. Image processing method according to Claim 22, wherein the graphical annotations of the annotated images having a similarity level above a determined similarity threshold are marked in a first graphical format in the result image, and wherein the graphical annotations of various images having a similarity level below the determined similarity threshold are marked in at least one second graphical format in the result image.
